# EUROPEAN PATENT SPECIFICATION

(11) **EP 1 605 959 B1**
(45) Date of publication and mention of the grant of the patent: **14.08.2013**
(21) Application number: 04709166.5
(22) Date of filing: 09.02.2004
(51) Int. Cl.: A61K 38/00, A61K 38/17, A61K 38/48, A61K 38/45, A61K 39/395

(54) **EPH/EPHRIN MEDIATED MODULATION OF CELL ADHESION AND TUMOUR CELL METASTASIS**
EPH/EPHRIN-VERMITTELTE MODULATION DER ZELLADHÄSION UND VON TUMORZELLMETASTASEN
MODULATION A MEDIATION EPH/EPHRINE DE L'ADHERENCE CELLULAIRE ET DES METASTASES CELLULAIRES TUMORALES

(30) Priority: 07.02.2003 AU 2003900541
(43) Date of publication of application: 21.12.2005
(73) Proprietor: Ludwig Institute for Cancer Research Ltd., New York, NY 10017 (US); MONASH UNIVERSITY, Clayton Victoria 3168 (AU); THE COUNCIL OF THE QUEENSLAND INSTITUTE OF MEDICAL RESEARCH, Brisbane, QLD 4029 (AU); THE UNIVERSITY OF QUEENSLAND, St Lucia, QLD 4072 (AU)
(72) Inventor: LACKMANN, Martin, Parkville, VIC 3050 (AU); WIMMER-KLEIKAMP, Sabine, Parkville, VIC 3050 (AU); SCOTT, Andrew, Parkville, VIC 3050 (AU); VEARING, Christopher, Parkville, VIC 3050 (AU); BOYD, Andrew, Herston, QLD 4029 (AU)
(74) Representative: Brasnett, Adrian Hugh
(86) International application number: PCT/AU2004/000142
(87) International publication number: WO 2004/069264

(56) References cited:
- WO-A1-00/30673
- WO-A1-01/12172
- WO-A1-02/26827
- WO-A1-99/08696
- WO-A1-03/004057
- WO-A1-03/099313
- WO-A2-02/058538
- LACKMANN MARTIN ET AL: "Expression and function in modulating tumor cell-cell contacts identifies EphA3 as candidate cell-surface receptor for tumor targeting strategies" PROCEEDINGS OF THE ANNUAL MEETING OF THE AMERICAN ASSOCIATION FOR CANCER RESEARCH, NEW YORK, NY, US, vol. 45, 27 March 2004 (2004-03-27), page 1015, XP001538538 ISSN: 0197-016X
- VEARING CHRISTOPHER ET AL: "Concurrent binding of anti-EphA3 antibody and ephrin-A5 amplifies EphA3 signaling and downstream responses: Potential as EphA3-specific tumor-targeting reagents" CANCER RESEARCH, AMERICAN ASSOCIATION FOR CANCER RESEARCH, BALTIMORE, MD., US, vol. 65, no. 15, 1 August 2005 (2005-08-01), pages 6745-6754, XP002493162 ISSN: 0008-5472
- LAWRENSON I.D. ET AL.: 'Ephrin-A5 induces roundig, blebbing and de-adhesion of EphA3- expression 293T and melanoma cells by CrkII and Rho-mediated signalling' J. CELL. SCIENCE vol. 115, 2002, pages 1059 - 1072, XP008102347
- WALKER-DANIELS J. ET AL.: 'c-Cbl-dependent EphA2 protein degradation is induced by ligand binding' MOL. CANCER RES. vol. 1, 2002, pages 79 - 87, XP002309751
- MARME D.: 'VEGFs, angiopoietins, ephrins and their receptors : putative targets for tumor binding' ANNALS OF HEMATOLOGY vol. 81, no. SUPPL 2, 2002, page 66, XP008100009
- KOOLPE M. ET AL.: 'An ephrin mimetic peptide that selectively targets the EphA2 receptor' J. BIOL. CHEM. vol. 277, no. 49, 2002, pages 46974 - 79, XP002309754

## Description

### FIELD OF THE INVENTION

THIS INVENTION relates to modulation of cell adhesion and cell repulsion. More particularly, this invention relates to modulation of cell adhesion and cell contact repulsion in response to ephrin binding by cells that express Eph receptors. A particular feature of the present invention is that cell repulsion and cell adhesion are triggered by distinct, cell type and Eph kinase activity-dependent pathways in response to ephrin binding. Accordingly, this invention particularly relates to preventing, inhibiting or delaying tumour cell metastasis through modulation of Eph receptor-ephrin binding interactions and subsequent Eph receptor internalization and signalling.

### BACKGROUND OF THE INVENTION

Eph receptors and their membrane- bound ephrin ligands act as cell guidance cues that co-ordinate the movement of cells and cell layers by mediating repulsive or adhesive signals (Boyd and Lackmann, 2001). Cell contact-dependent, ephrin-induced cell-cell repulsion relies on both, signals from the active receptor tyrosine kinase (Lawrenson et al, 2002), and regulated proteolytic ligand cleavage to disrupt the high-affinity, multivalent receptor/ligand interactions (Hattori et al., 2000). Expression of EphA splice variants lacking the kinase domain during mouse development can shift cellular responses to the same receptor from contact repulsion to cell-cell adhesion (Holmberg et al., 2000). It is now clear from these and other studies that in the absence of cytoplasmic Eph receptor signalling function and lack of cleavage of the Eph/ephrin tether (Hattori et al., 2000) cell contact repulsion switches to cell-cell adhesion. In addition, Eph receptor activation can augment cell-substrate adhesion (Holmberg and Frisen, 2002) by crosstalk to áᵥâ₃ or á₅â₁ integrins, increasing their affinity for their ligands vitronectin or fibronectin (Huynh-Do et al.,1999, Becker et al., 2000). Analysis of Eph and ephrin mutants during different developmental processes in *C-elegans* and mouse has emphasised the importance of Eph/ephrin mediated cell repulsion, cell adhesion, as well as kinase-dependent and kinase-independent Eph signalling (George et al., 1998, Wang et al, 1999,Birgbauer et al., 2001, Kullander et al., 2001, Birgbauer et al., 2000).

There is little indication for Eph receptor/ephrin function in normal adult tissue, but increasing evidence implies that these families of molecules are involved in cancer progression and tumour neovascularisation (Dodelet and Pasquale, 2000, Ogawa et al., 2000). However, in contrast to the well-defined developmental roles of Eph receptors and ephrins, their function in cancer cell biology is only beginning to be explored (Batlle et al, 2002). EphA3, originally isolated as antigen on the surface of LIC63 lymphoblastic pre-B cells (Boyd et al., 1992), is over-expressed in several tumours, including lung cancer, neuroblastoma, brain and renal tumours and melanoma (Wang and Anderson, 1997, Wicks et al., 1992, Chiari et al, 2000). Recently, EphA3 was re-discovered as tumour antigen involved in a tumour rejection response of a melanoma patient (Chiari et al., 2000).

Lawrenson et al., J Cell Science (2002) 115(5):1059-1072 describes that Eph receptors and ephrins are overexpressed in a range of tumours and that EphA3 activation induces retraction of cell protrusions, cell rounding, membrane blebbing and de-adhesion and that ephrin A5-mediated receptor clustering and tyrosine kinase activity of EphA3 are essential for this response.

### SUMMARY OF THE INVENTION

The present inventors have realised the need to better understand the functional significance of Eph receptor expression and Eph-ephrin interactions across the increasingly diverse tumour cell types that express Eph receptors.

Surprisingly, the present inventors propose that either cell adhesion or cell contact repulsion occur in response to ephrin binding, the particular response being dependent on the type of tumour cell that expresses the Eph receptor.

The invention is therefore broadly directed to the modulation of cell adhesion, cell-contact repulsion, invasion and/or metastatsis by modulation of the Eph receptor-ephrin system.

In one aspect, the invention provides an antibody to an ephrin-interacting domain of an EphA3 receptor, for use in preventing, delaying or inhibiting tumour cell metastasis by modulating the ability of a tumour cell expressing said EphA3 receptor to respond to ephrin binding in a mammal, whereby cell-contact repulsion between said tumour cell and another cell that expresses said ephrin is enhanced.

In a second aspect is provided a pharmaceutical composition comprising an antibody directed to an ephrin-interacting domain of an EphA3 receptor, for use in preventing, delaying or inhibiting tumour cell metastasis by modulating the ability of a tumour cell expressing said EphA3 receptor to respond to ephrin binding in a mammal, whereby cell-contact repulsion between said tumour cell and another cell that expresses said ephrin is enhanced.

An example of a tumour cell according to this embodiment is a malignant melanoma cell or a kidney tumour cell. Experimental models of such cell types are LiBr melanoma cells or human epithelial kidney (HEK) 293 cells.

Where cell-cell adhesion is to be inhibited or reduced, the tumour cell normally responds to ephrin binding by adhesion to another cell that expresses the bound ephrin.

An example of such a tumour cell is a lymphoblastic tumour cell, such as a pre-B leukaemia cell. An experimental model of such a cell type is LK63.

Preferably, the Eph receptor is EphA3.

Preferably, the ephrin is ephrin A5.

Also described herein is a method of identifying an agent that modulates cell adhesion and/or cell repulsion, said method including the step of determining whether said agent modulates cell adhesion or cell repulsion in response to ephrin binding.

Also described herein is a pharmaceutical composition that comprises an agent for use in modulating Eph receptor-ephrin mediated cell adhesion, together with a pharmaceutically-acceptable carrier diluent or excipient.

Also described is the use of an agent that modulates Eph receptor/ephrin mediated cell adhesion and contact repulsion by specifically targeting Eph receptor-expressing tumor cells and through internalisation into the lysosomes of these cells and release of a grafted cytotoxic drug in the acid environment will kill the targeted tumor cell.

Throughout this specification, unless otherwise indicated, "comprise", "comprises" and "comprising" are used inclusively rather than exclusively, so that a stated integer or group of integers may include one or more other non-stated integers or groups of integers.

### BRIEF DESCRIPTION OF THE FIGURES

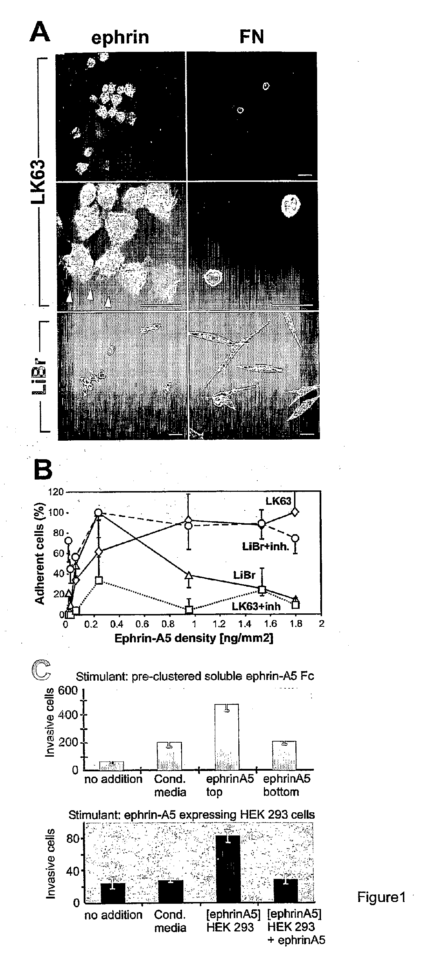

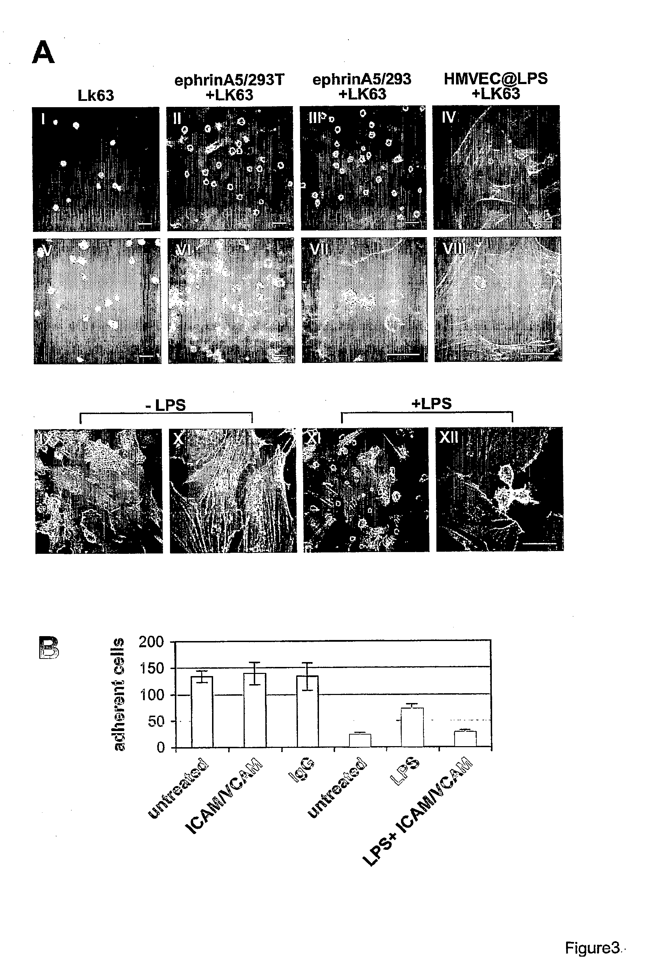

Figure 1. Surface-bound ephrinA5 causes either cell repulsion or cell adhesion of EphA3-positive tumour cells.
   (A) Eph-A3-positiveLK63 human pre-B leukemia cells and LiBr melanoma cells were plated onto glass coverslips with ephrin-A5-Fc (ephrin, 10 µg/ml) or fibronectin (FN, 10 µg/ml). After 4h the cytoskeleton of adherent, fixed cells was stained with rhodamin-phalloidin. Pictures of representative fluorescence images are shown; the second column represents magnified sections of the first. Scale bar: 20 i m.
   (B) LK63 adhesion and LiBr de-adhesion to surface-bound ephrinA5 are dose-dependent. LK63 (2x10⁵ cells/well) and LiBr cells (5x10⁴ cells/well) were seeded into wells of protein A-grafted 96-well culture plates that had been coated with ephrinA5-Fc at indicated densities. Soluble, monomeric ephrinA5 was added as inhibitor (+ inhibitor) to parallel LK63 and LiBr cultures at 100-fold molar excess prior to seeding. After 4-hour incubation, adherent cells, withstanding rigorous washing, were quantitated by XTT assay (A₄₉₂ absorbance). Cell attachment is expressed as a percentage (mean, S.E.. from three independent assays) relative to wells containing most adherent cells; ( ), LK63 and ( ), LiBr cells and ( ), LK63, ( ),LiBr cells with ephrin inhibition (+inh.).
   (C) LiBr melanoma cells in matrigel-coated Basement Membrane migration chambers (Becton Dickinson, 5x10⁴/well ) were exposed to chemoattractant, 3T3 conditioned media (cond. media) or 1.5 i g/ml pre-clustered ephrin-A5 Fc in the bottom chamber, or with 1.5 ì g/ml pre-clustered ephrin-A5 Fc placed together with cells into the top chamber. After 6-7h, non-invaded cells from the top of the occluding membrane (8ì m) were removed, cells that had passed through the pores were stained (Diff-Quick, Fisher Scientific) and counted. 15 Mean cell numbers from 3 independent experiments are shown (top panel). Alternatively, LiBr cells that had been stained with Cell Tracker Green CMFDA (Molecular Probes) were incubated together with ephrin-A5/HEK 293 cells (each, 5x10⁴/well) in the top chamber and invasive cells on the underside of the membrane counted under the fluorescence microscope. To inhibit the cell-contact repulsion between ephrin-A5/HEK 293 cells and LiBr cells, soluble ephrin-A5 Fc (non-clustered) was added into the top chamber as indicated.

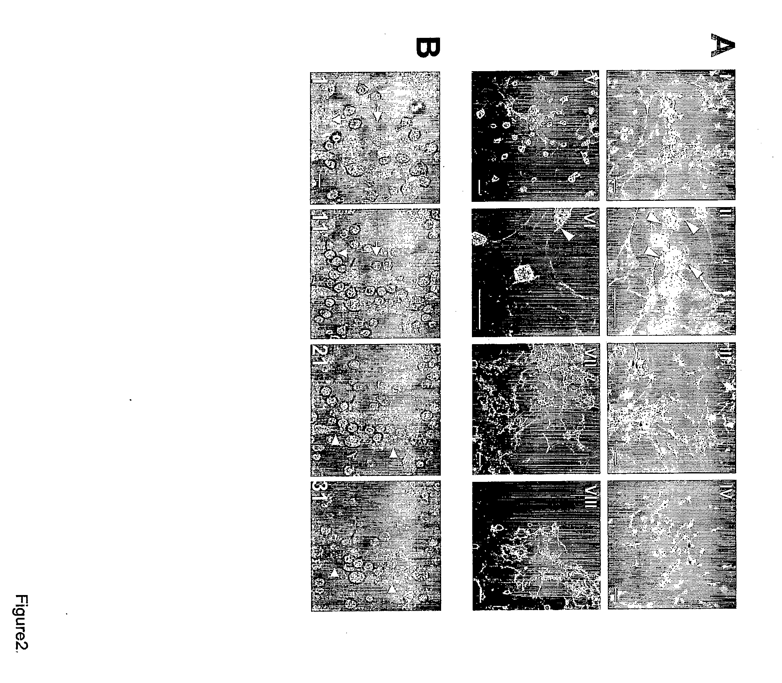

Figure 2. EphA3/ephrinA5-facilitated cell-cell adhesion.
   A) LK63 pre B leukemia cells were added to cultures of ephrinA5-positive mouse d.14.5-cortical neurons (panels I-III), ephrinA5/HEK 293 cells (panels 25 V-VII) or parental HEK293 cells (panel VIII), grown on fibronectin-coated glass coverslips. To disrupt EphA3/ephrinA5 interactions, soluble monomeric ephrin5 was added to some cultures (panel III, VII) at 100-fold molar excess. Non-adherent cells were removed and remaining adherent cells stained with rhodamin-phalloidin. Anti DCC (I-III) and anti ephrinA5 30 antibodies (IV) were used to stain d.14.5-cortical neurons (IV) and endogenous ephrinA5, respectively. Panels II and VI represent magnified sections of panels I and V. Scale bar: 20 i m.
   B) EphrinA5/HEK 293 cells on fibronectin-coated coverslips were incubated with limiting amounts of Alexa EphA3-Fc to visualise cell-surface ephrinA5. Following addition of LK63 cells to the washed ephrinA5/HEK 293 monolayers, selected fluorescent (red) and light microscopic images (grey) taken at 20 see intervals in a 32min time-course are merged for this representation. Scale bar: 20 ìm.
f Figure 3. EphA3-mediated LK63 cell adhesion is independent of VCAM and ICAM interactions.
   A) LK63 cells were added to EphrinA5/HEK 293 cells (II, III, VI, VII) and HMVECs (IV, VIII - XII), in the absence (IX-XII) or presence of function blocking á-VCAM (II) and á-ICAM (VI) antibodies alone, or in combination (III, IV, VII, VIII). After 60-min, the actin cytoskeleton of cells remaining attached and cell-bound á-VCAM and á-ICAM antibodies were stained with rhodamin-phalloidin (II-IV, VI-VIII, IX-XII) and with secondary Alexa 488-conjugated antibodies (III, IV, VII, VIII), respectively. Merged microscopic images (Alexa, green; rhodamin, red) are shown (II-IV, VI-VIII). VCAM 15 and ICAM expression on fixed LK63 cells was examined with á-VCAM (I) and á-ICAM (V) antibodies. EphA3-independent adhesion of LK63 cells to LPS-treated (panels XI, XII, +LPS), or untreated HMVECs (panels IX, X, - LPS) was analysed in parallel. Details in panels III, IV, IX, XI are shown at 2-fold magnifications (VII, VIII, X, XII). Scale bar: 20 ì m.
   B) Cell-cell adhesion was quantitated by counting LK63 cells remaining attached to untreated, á-ICAM-1/á-VCAM or control IgG treated, ephrin-A5/293 cells ( ) or untreated, LPS or LPS and á-ICAM-1/á-VCAM treated HMVECs ( ) in a minimum of four representative microscopic sections at 10x magnification. Mean cell number and S.E. are shown.

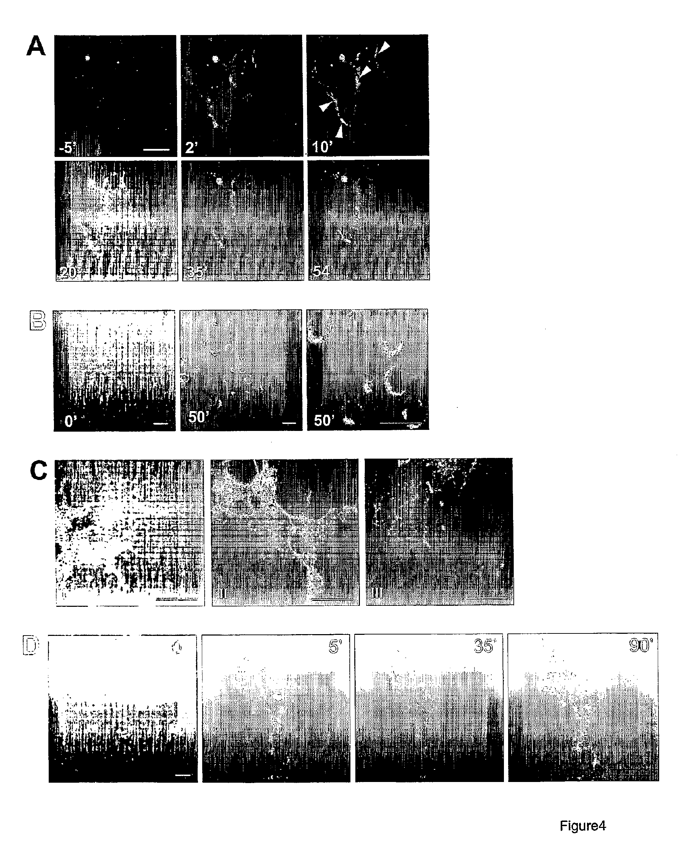

**Figure 4****.** Eph/ephrin mediated repulsion, but not adhesion, leads to ephrinA5 intemalisation.
   (A) EphA3/HEK 293 cells on fibronectin coated glass coverslips were stimulated with pre-clustered Alexa ephrinA5-Fc. Fluorescent confocal microscopic images represent selected time points during a 60-min. time course, starting 5 min before ephrin addition. Scale bar: 20 i m.
   (B) In a parallel experiment LK63 cells were treated as described in (A) and analysed for Alexa ephrin5 internalisation by confocal microscopy. Scale bar: 20 i m.
   (C) EphA3/HEK 293 cells were treated with pre-clustered Alexa ephrinA5-Fc in the absence (I) or in the presence of 'Fc block' (II), or with non-clustered Alexa ephrinA5-Fc (III). Fixed cells after 30-min stimulation were mounted onto slides and analysed by confocal fluorescence microscopy.
   (D) Prior to stimulation with pre-clustered Alexa ephrinA5-Fc, the lysosomal compartments of EphA3/HEK 293 cells were stained with Lysotracker™ green and the translocation of receptor/ligand complexes to the lysosomes monitored by confocal time-lapse microscopy, carried out sequentially at two excitation wavelengths. The resulting green (Lysotracker™) and red (Alexa Fluor 546) images at indicated time points were merged.

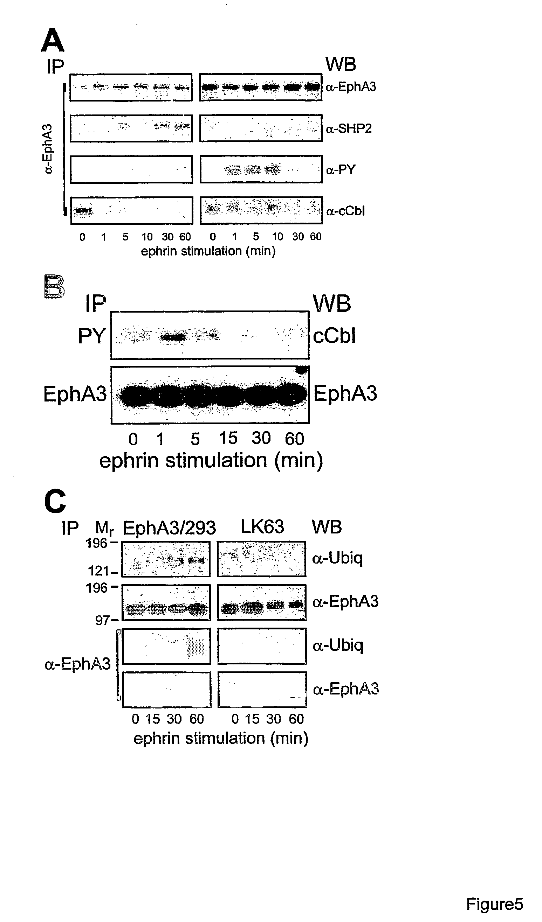

Figure 5. EphA3 mediated cell adhesion and repulsion involve distinct biochemical pathways.
   (A) Anti-EphA3 immunoprecipitates from Triton-X100 lysates of EphA3/ HEK 293 (right column) or LK63 cells (left column), treated for indicated 15 times with pre-clustered ephrinA5-Fc, were analysed by Western blot with anti- EphA3, anti-phosphotyrosine, anti c-Cbl and anti SHP2 antibodies as indicated.
   (B) Ephrin-A5-induced phosphorylation of c-Cbl was analysed in anti-phophotyrosine immunorecipitates from Triton-X100 lysates of EphA3/HEK 20 293 cells treated for indicated times with pre-clustered ephrinA5, using anti-EphA3 and anti c-Cbl antibodies as indicated.
   (C) Stimulation of EphA3/HEK 293 cells but not of LK63 cells results in ubiquitination of EphA3 on the plasma membrane. Plasma membrane fractions or derived anti-EphA3 immunoprecipitates of ephrinA5 stimulated 25 EphA3/HEK 293 cells or of LK63 cells were analysed in Western Blots with antibodies against EphA3 and ubiquitin as indicated.

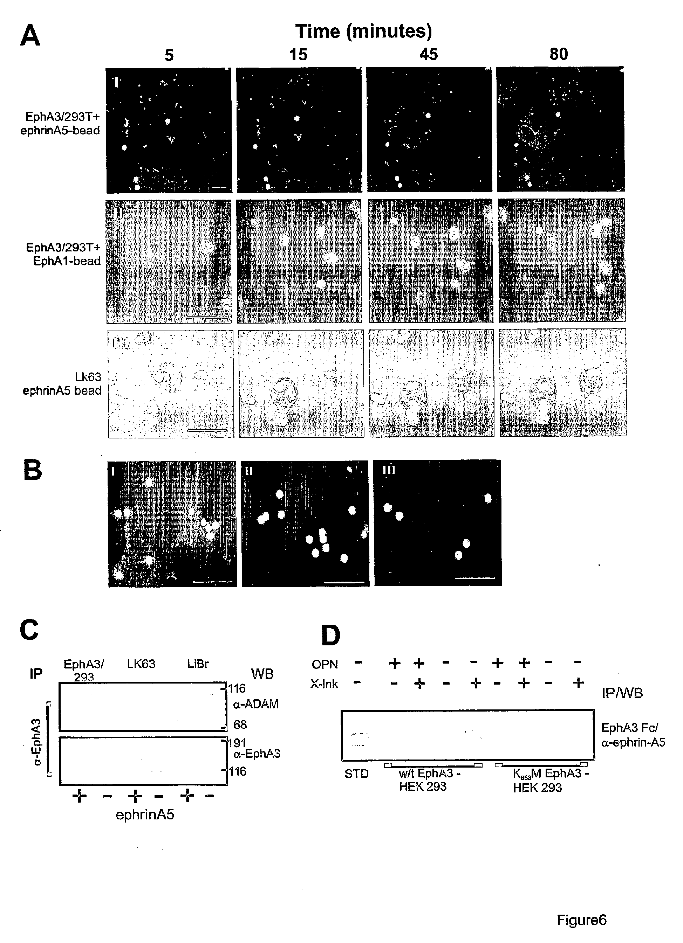

Figure 6. Eph/ephrin mediated cell-cell repulsion, but not adhesion leads to ephrin5 cleavage by a metalloprotease.
   (A) Alexa ephrinA5-Fc (I, III) or Alexa EphA1 Fc (II) control proteins, 30 conjugated onto protein A-coated Dynabeads were added to cultures of EphA3/HEK 293 cells (I, II) or LK63 cells (III). Cleavage and internalisation of the fluorescent proteins was monitored by confocal microscopy. Selected images of confocal time-lapse experiments (1 frame/min) are shown. Alexa 546 fluorescence is represented in white and outlines cells in panels I, while the cells in panels II, III appear dark-grey or black.
   (B) Prior to stimulation (30min) with Alexa ephrinA5-Fc coated beads (I, II) or with Alexa Fc control beads (III), parallel cultures of EphA3/293 cells 5 were treated for 4h with 5mM 1',10'-O-Phenanthroline (II) or left untreated (I, III). Cells, fixed in 4% PFA, were analysed for cleavage and internalisation of the labelled proteins by confocal fluorescence microscopy.
   (C) Anti-EphA3 immunoprecipitates from non-stimulated (-) or ephrinA5-stimulated (+) LK63, LiBr or EphA3/293 cells were analysed by Western blot with anti-ADAM10 and anti-EphA3 antibodies as indicated.
   *(D)* Biochemical demonstration of ephrin cleavage. Using an EphA3-Fc construct, ephrin-A5 was immuno precipitated from lysates of EphA3/HEK 293 cells or LK63 cells, which had been treated for indicated times with 7.5 i g/ml of pre-clustered (+ X-lnk) or non-clustered (-X-lnk) ephrin-A5 Fc, and 15 analysed for ephrin cleavage by Western blot with anti- ephrin5 antibody. To assess cleavage by metalloproteases, parallel cultures were treated with 1',10'-O-phenanthroline (+ OPN) or left untreated.
Figure 7. Pervanadate-induced Eph receptor phosphorylation HEK293 cells, expressing w/t EphA3GFP (A) or 3YF EphA3GFP (B) were 20 incubated with pervanadate (30 min) at indicated concentrations. Fixed cells were examined for FRET as described above. Left, GFP-fluorescence; right, GFP fluorescence lifetime phase maps. Tabulated colour codes indicate GFP lifetimes in ns.
Figure 8. Phosphatase inhibition triggers EphA3 phosphorylation in LK63 25 cells
   (A) Anti-EphA3 immunoprecipitates from TritonX100 lysates of EphA3/ HEK 293, EphA3/AO2 melanoma cells and LK63 leukaemia cells were treated with either crosslinked ephrin-A5 or increasing concentrations of sodium pervanadate as indicated, and analysed by anti-phosphotyrosine western blot.
   (B) Anti-EphA3 immunoprecipitates from TritonX100 lysates of vanadate or hydrogen peroxide-treated EphA3/ HEK 293 (left panel) and EphA3/AO2 melanoma cells(right panel) were analysed as described in (A).

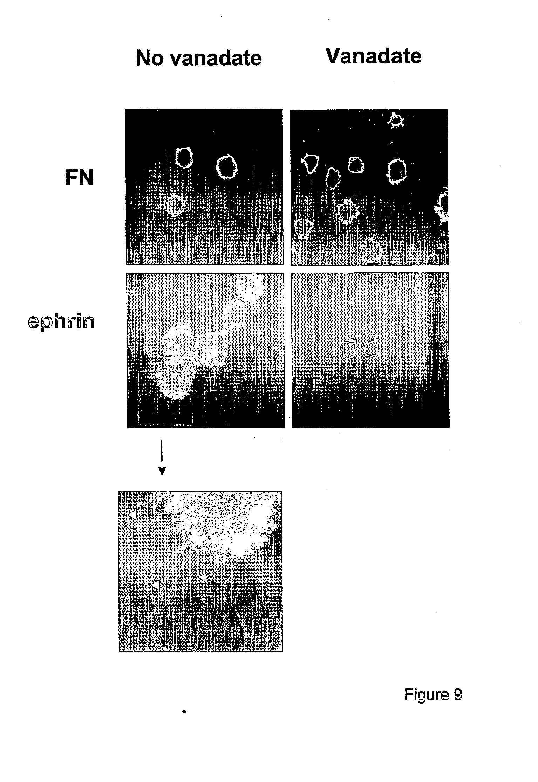

Figure 9. Phosphatase inhibition abrogates ephrin-mediated cell adhesion. LK63 cells, seeded onto ephrin- or FN coated glass coverlips as described in Figure 1A were treated with vanadate (vanadate) or left untreated (no vanadate). After 4h the cytoskeleton of adherent, fixed cells was stained 5 with rhodamine-phalloidin. Pictures of representative fluorescence microscopic images are shown. Scale bar: 20 i m.

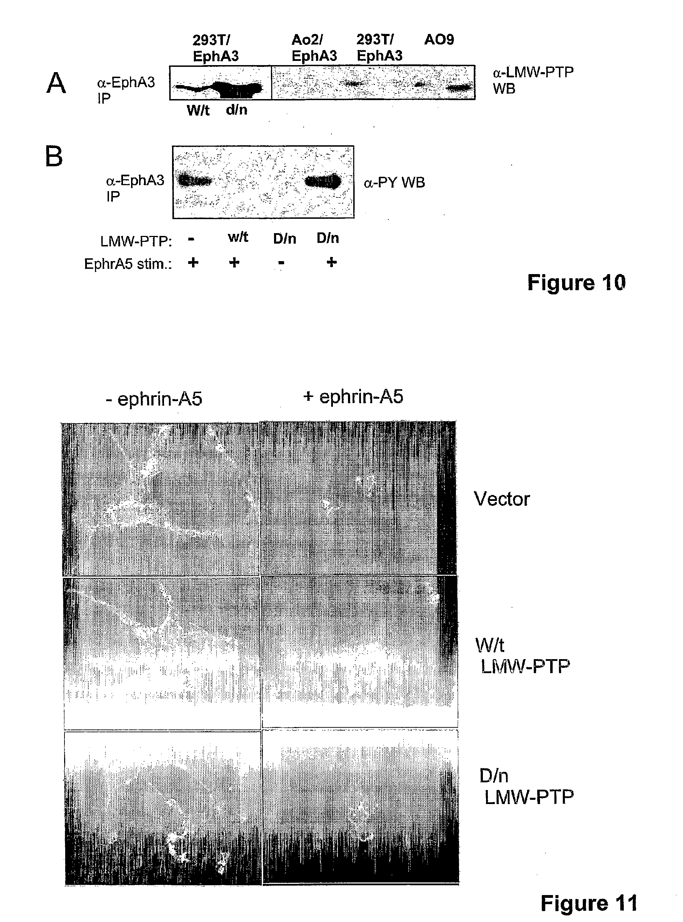

Figure 10. LMW-PTP modulates EphA3 phosphorylation
   (A) Association of endogenous and recombinant LMW-PTP with EphA3. Anti-EphA3 immunoprecipitates of EphA3 expressing EphA3/HEK 293cells, EphA3/ AO2 and AO9 melanoma cell lysates, or lysates of EphA3/HEK 293 cells transiently transfected with w/t or dominant negative (d/n) LMW-PTP were analysed with anti-LMW-PTP western blot.
   (B) EphA3/ 293T cells were transiently transfected with w/t or d/n LMW-PTP. Anti-EphA3 immunoprecipitates from TritonX100 lysates of cells stimulated with crosslinked ephrin-A5 were analysed by anti-phosphotyrosine Western blot.
Figure 11. LMW-PTP modulates EphA3 -mediated cell-morphology changes. EphA3/HEK 293 cells were transiently transfected with empty vector or cDNAs encoding w/t or d/n LMW-PTP as indicated. Non-stimulated or (pre-clustered) ephrin-A5 Fc stimulated cells on fibronectin-coated coverslips were analysed by Alexa-Phaloidin staining for cytoskeletal changes by confocal microscopy.

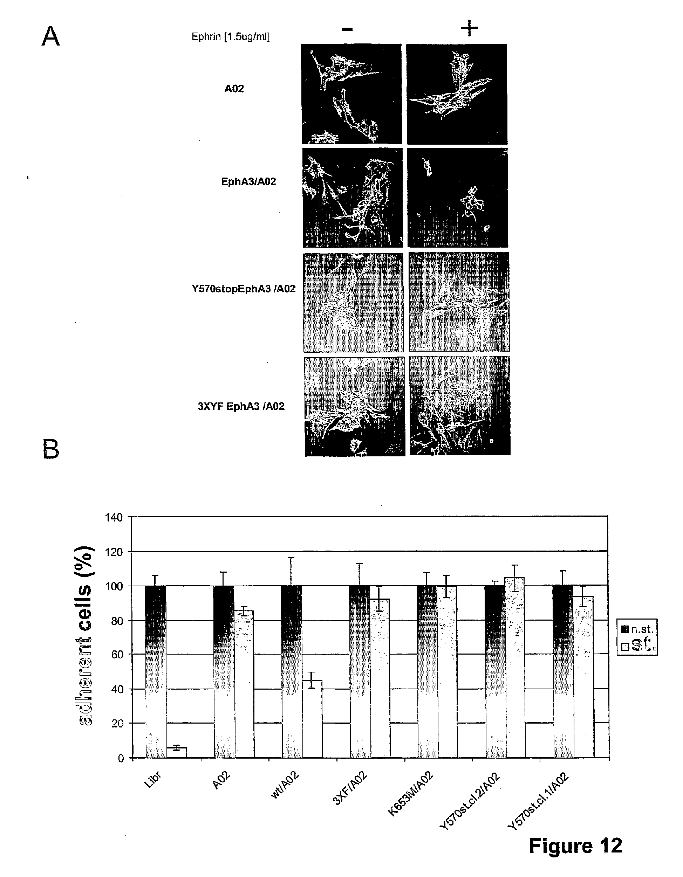

Figure 12. EphA3 kinase activity is essential for the repulsion response
   (A) AO2 melanoma cells, stably transfected with EphA3 w/t, 3XYF/EphA3, K570Stop/EphA3, K653M/EphA3 or non-transfected parental cells on fibronectin-coated glass coverslips were stimulated with 10 nM clustered ephrin-A5 Fc, while parallel cultures were left non-stimulated. Fixed and Alexa 488 phalloidin stained samples were analysed for their cell 30 cytoskeletal characteristics by confocal microscopy.
   (B) Melanoma cells grown for at least 4h at defined densities on fibronectin coated 96-well plates were ephrin-A5- stimulated as described in (A). Adherent cells, withstanding rigorous washing, were quantitated by XTT assay (A₄₉₂ absorbance). Cell attachment is expressed as a percentage
(mean, S.E. from quadruplicate wells) relative to wells containing most adherent cells.

### DETAILED DESCRIPTION OF THE INVENTION

The present invention arises, at least in part, from the present inventors' comparison of cell biological and biochemical responses of different EphA3 expressing cancer cells to ephrinA5. LK63 leukemia cells normally grow in suspension but adhere to surface-tethered ephrinA5 and ephrinA5 expressing cells and undergo dramatic cell morphological changes. Surprisingly, EphA3 activation of LiBr melanoma cells induces the retraction of cell protrusions, cell rounding and de-adhesion. More particularly, cell repulsion entails rapid, metalloprotease-mediated ephrin-cleavage and intemalisation, pronounced phosphorylation of EphA3 and c-Cbl and EphA3-ubiquitination. However, little or no ephrin cleavage is observed in the absence of clustering or in EphA3-kinase-defect cells and no ephrinA5 cleavage or EphA3/ephrinA5 internalisation is observed in LK63 cells, which display only marginal EphA3 phosphorylation and recruit the tyrosine phosphatase SHP-2 upon ephrin-A5 exposure. Thus, cell repulsion and adhesion as well as ephrinA5 cleavage and internalisation are specific for the Eph/ephrin interaction and rely on artificially clustered or surface-bound ephrinA5. They can thus be inhibited competitively with non-clustered ephrinA5. Disparate signalling pathways from the same Eph receptor command either cell-cell repulsion or adhesion of cancer cells. While cell repulsion is an important mechanism for cell dislodgement from the primary site, a reversal of EphA3 function from cell repulsion to cell adhesion may provide a docking mechanism for metastasising tumour cells.

As will be appreciated from the foregoing, the invention contemplates modulation of cell repulsion between cells that respectively express an Eph receptor or an ephrin. Accordingly, it will be understood that typically, these cells express an *"endogenous"* ephrin or Eph receptor, although it is also possible that these cells could be engineered to express a recombinant Eph receptor or ephrin not normally expressed by the cell(s).

Accordingly, contemplated is the use of an agent in the form of an *"exogenous"* ephrin and/or Eph receptor, typically although not exclusively a recombinant protein in soluble form and, in certain embodiments, recombinant as a fusion protein with another molecule such as an Fc portion of an antibody conjugated to a cytotoxic drug or a radioisotope.

The present invention is therefore broadly directed to manipulation of Eph receptor-ephrin interactions and downstream signalling, such as associated with proteolytic cleavage, internalisation of Eph receptor-bound ephrin and Eph receptor-mediated phosphorylation, to thereby modulate cell repulsion and tumour metastasis. This can be achieved in particular by protein-protein interaction inhibitors, preferably non-clustered ephrin itself. Furthermore, by targeting cells expressing ephrin-A5 interactive Eph receptors (EphA2 - 5, EphA7, 8, EphB2) provided is a method that selectively kills these cells by release of a hydrolysable ephrin-A5 conjugated cytotoxic drug upon Eph-receptor-mediated internalisation of such a conjugate.

In light of the foregoing, it will be appreciated that tumour cell metastasis may be manipulated on various levels, including tumour cell spreading from the original site, colonisation of new tumor sites and neovascularisation according to the cell type concerned.

For example, administration of an agent such as a soluble ephrin (for example in the form of ephrin-A5 or an ephrin-A5/human Fc fusion protein, ephrin-A5-Fc) as a competitive inhibitor, or ephrin-A5-Fc conjugated through an acid-labile bond to a cytotoxic reagent (such as hydrolysable calicheamicin, (Hamann et al. 2002)) may reduce or inhibit LK63 cell attachment to ephrin-expressing cells or induce LK63 cell death.

Although not wishing to be bound by any particular theory, it is possible that also de-adhesion of tumour cells may lead to tumour cell apoptosis.

Conversely, administration of an agent such as a soluble ephrin or souble Eph receptor ligand-binding domain, for example in the form of soluble ephrin-A5-Fc or soluble EphA3-Fc, may reduce or inhibit LiBr melanoma cell-cell repulsion.

It will also be appreciated that an agent such as "clustered", "aggregated" or "surface anchored" ephrin is contemplated, for use in inducing cell repulsion and to enhance its uptake into EphA3-expressing tumour cells.

Also described is the use of an antibody directed to an ephrin to block, inhibit or reduce adhesion between the cell expressing the Eph receptor and said another cell expressing said ephrin.

The invention contemplates use of an antibody directed to an ephrin interaction or binding domain of an Eph receptor to enhance repulsion between the cell expressing the Eph receptor and said another cell expressing said ephrin.

For example, in a particular embodiment, the invention contemplates the use of an antibody directed to an ephrinA5 interaction domain or said Eph receptor.

This embodiment is supported by the surprising observation that exposure of HEK 293 cells to ephrin-A5 and the anti-Eph A3 mAb IIIA4, in combination, results in an enhanced cell-morphological response leading to pronounced rounding and detachment. Furthermore, EphA3 tyrosine phosphorylation levels in IIIA4-treated or in ephrin-A stimulated cells increased dose and time-dependent but were amplified dramatically in cells treated with non-clustered or with preclustered IIIA4 in combination with ephrin-A5 Fc (data not shown).

Further agents include molecules that prevent the association of proteases with EphA3 or ephrin-A5 or inhibit metalloprotease activity associated with ephrin cleavage, internalisation and cell repulsion.

In particular cases, the invention contemplates the use of inhibitors of ADAM10 and/or related metalloproteases.

Also described are agents that regulate intracellular tyrosine phosphorylation, and more particularly Eph receptor phosphorylation, to modulate cell adhesion. As will be described in more detail hereinafter, Eph-receptor mediated cell-cell adhesion appears to result from down-modulated Eph receptor tyrosine kinase activity.

A particular example of such an agent is an inhibitor of protein tyrosine phosphatase activity to thereby increase Eph receptor tyrosine phosphorylationi and promote contact repulsion or detachment of cells that would adhere to ephrin expressing cells.

Described are specific inhibitors of the protein tyrosine phosphatase SHP-2, LMWPTP and/or related protein tyrosine phosphatases.

In addition, described are hydrolysable fusion proteins between ephrin-A5 and a cytotoxic drug such as calicheamicin that will specifically induce cell killing upon Eph-receptor-mediated ephrin-A5 internalisation and translocation into lysosomes.

Also described are derivatives of ephrin-A5 conjugated to radiometals such as ¹¹¹In or ⁹⁰Y that will induce cell killing upon Eph-receptor-mediated ephrin-A5 internalisation.

Still further agents include ephrin mutants, agonists, analogues, antagonists, antibodies and mimetics that are produced or engineered for use in modulating cell adhesion and/or cell repulsion by targeting Eph receptor-ephrin binding interactions and or intracellular signalling specifically associated with cell adhesion and/or cell repulsion.

The aforementioned mimetics, agonists, antagonists, ephrin-derived cytotoxic tumor targeting reagents and analogues may be peptides, polypeptides or other organic molecules, preferably small organic molecules, with a desired biological activity or half-life.

With regard to mutant ephrins, these may be created by mutagenising wild-type protein, or by mutagenising an encoding nucleic acid, such as by random mutagenesis or site-directed mutagenesis. Examples of nucleic acid mutagenesis methods are provided in Chapter 9 of CURRENT PROTOCOLS IN MOLECULAR BIOLOGY, Ausubel et al., *supra.*

Random mutagenesis methods include chemical modification of proteins by hydroxylamine (Ruan et al 1997, Gene 188 35), incorporation of dNTP analogs into nucleic acids (Zaccolo et al., 1996, J. Mol, Biol, 255 589) and PCR-based random mutagenesis such as described in Stemmer, 1994, Proc. Natl, Acad. Sci. USA 91 10747 or Shafikhani et al., 1997, Biotechniques 23 304. It is also noted that PCR-based random mutagenesis kits are commercially available, such as the Diversify™ kit (Clontech).

Eph-modulating agents of the invention may also be identified by way of screening libraries of molecules such as synthetic chemical libraries, including combinatorial libraries, by methods such as described in Nestler & Liu, 1998, Comb. Chem. High Throughput Screen. 1 113 and Kirkpatrick et al., 1999, Comb. Chem. High Throughput Screen 2 211.

It is also contemplated that libraries of naturally-occurring molecules may be screened by methodology such as reviewed in Kolb, 1998, Prog. Drug. Res. 51 185.

An alternative approach is to utilize computer-assisted structural database searching, such as for identifying and designing ephrin mimetics. Database searching methods which, in principle, may be suitable for identifying mimetics, may be found in International Publication WO 94/18232 (directed to producing HIV antigen mimetics). United States Patent No. 5,752,019 and International Publication WO 97/41526 (directed to identifying EPO mimetics).

Other methods include a variety of biophysical techniques which identify molecular interactions, such as competitive radioligand binding assays, analytical ultracentrifugation, microcalorimetry, surface plasmon resonance and optical biosensor-based methods. Examples of these methods are provided in Chapter 20 of CURRENT PROTOCOLS IN PROTEIN SCIENCE Eds. Coligan et al., (John Wiley & Sons, 1997) which is incorporated herein by reference.

It will be apparent that the present invention is not limited to the aforementioned embodiments that have been exemplified herein. The present invention provides a new principle, namely that Eph receptor-ephrin binding interactions may be manipulated in a cell type-specific manner to thereby affect cell migration and repulsion.

With regard to tumour cell metastasis, the present invention is applicable to any tumour cell type where the Eph receptor-ephrin mediates cell adhesion and/or cell repulsion. Non-limiting examples of such tumour cells include leukemias and lymphomas, lung and colon cancer, neuroblastoma, brain, renal and kidney tumours, prostate cancers, sarcomas and melanoma.

For a more comprehensive review of potentially relevant tumours the skilled person is directed to Nakamoto & Bergemann, 2002.

It will also be appreciated that although the present invention has been exemplified with respect to EphA3 and ephrin A5, the inventive principle set forth herein may apply to ephrin interactions with other Eph receptors, including EphB2, EphA2, EphA4, EphA5, EphA7, EphA8.

While particular emphasis has been placed on tumour cell metastasis, the present invention is generally applicable to the modulation of cell-cell communication mechanisms facilitating migration, adhesion and repulsion, such as for the purposes of tissue and nerve regeneration and patterning, wound healing, treatment of burns and ulcers and bone regeneration, for example.

The invention therefore provides pharmaceutical compositions that comprise an agent for use in modulating Eph receptor-ephrin mediated cell adhesion and/or repulsion.

Pharmaceutical compositions of the invention may be used to modulate cell migration, tissue regeneration and wound healing. Alternatively, pharmaceutical compositions may be administered to prevent or inhibit tumour metastasis.

The composition may be used in therapeutic or prophylactic treatments as required. For example, pharmaceutical compositions may be applied in the form of therapeutic or cosmetic preparations for skin repair, wound healing, healing of burns, bone regeneration and other dermatological treatments.

Suitably, the pharmaceutical composition comprises an appropriate pharmaceutically-acceptable carrier, diluent or excipient.

Preferably, the pharmaceutically-acceptable carrier, diluent or excipient is suitable for administration to mammals, and more preferably, to humans.

By *"pharmaceutically-acceptable carrier, diluent or excipient"* is meant a solid or liquid filler, diluent or encapsulating substance that may be safely used in systemic administration. Depending upon the particular route of administration, a variety of carriers, well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline and salts such as mineral acid salts including hydrochlorides, bromides and sulfates, organic acids such as acetates, propionates and malonates and pyrogen-free water.

A useful reference describing pharmaceutically acceptable carriers, diluents and excipients is Remington's Pharmaceutical Sciences (Mack Publishing Co. N.J. USA, 1991) which is incorporated herein by reference.

Any safe route of administration may be employed for providing a patient with the composition of the invention. For example, oral, rectal, parenteral, sublingual, buccal, intravenous, intra-articular, intra-muscular, intra-dermal, subcutaneous, inhalational, intraocular, intraperitoneal, intracerebroventricular, transdermal and the like may be employed.

Dosage forms include tablets, dispersions, suspensions, injections, solutions, syrups, troches, capsules, suppositories, aerosols, transdermal patches and the like. These dosage forms may also include injecting or implanting controlled releasing devices designed specifically for this purpose or other forms of implants modified to act additionally in this fashion. Controlled release of the therapeutic agent may be effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, the controlled release may be effected by using other polymer matrices, liposomes and/or microspheres.

The above compositions may be administered in a manner compatible with the dosage formulation, and in such amount as is pharmaceutically-effective. The dose administered to a patient, in the context of the present invention, should be sufficient to effect a beneficial response in a patient over an appropriate period of time. The quantity of agent(s) to be administered may depend on the subject to be treated inclusive of the age, sex, weight and general health condition thereof, factors that will depend on the judgement of the practitioner.

So that the present invention may be more readily understood and put into practical effect, the skilled person is referred to the following non-limiting examples.

### EXAMPLES

### Methods

### Expression constructs and reagents

Full-length ephrinA5 was cloned into a pEF BOS-derived mammalian expression vector containing a neomycin resistance cassette (pEF MC1neopA). The cloning of full length EphA3 (Wicks et al., 1992) into pEFBos (Nicola et al., 1996) has been described previously. Expression plasmids (pIgBOS) (Coulthard et al., 2001) encoding fusion proteins in which either the extracellular domains of ephrinA5 or EphA3 are fused to the hinge and Fc region of human IgG1 (gift from A. van der Merwe, Oxford University) were used to transfect Chinese Hamster Ovary (CHO) cells. EphA3-Fc and ephrinA5-Fc were purified from cell culture supernatants by protein-A affinity chromatography. Flag-tagged monomeric ephrinA5 was purified to homogeneity from transfected CHO cell supernatants as described previously (Lackmann et al., 1997).

A native EphA3 specific (clone IIIA4) monoclonal antibody (Mab) and affinity-purified rabbit polyclonal antibodies have been described previously (Boyd et al., 1992; Lackmann et al., 1997). Additional antibodies and reagents were purchased from Transduction Laboratories (á-c-Cbl), New England Biolabs (á-phosphotyrosine), Santa Cruz (á-ephrinA5, á-SHP-2, á-ubiquitin), and Biogenesis (á-ADAM 10). HRP-labelled secondary antibodies were from Jackson laboratories (anti-mouse) and BioRad (anti-rabbit). Alexa-labelled secondary antibodies, rhodamin-phalloidin and lysotracker (green) were purchased from Molecular Probes. Mouse anti human ICAM-1 and VCAM-1 MAbs were generous gifts from Ian Wicks (The Walter & Eliza Hall Institute, Melbourne). Lipopolysaccharide (LPS) was obtained from Sigma.

### Cell Culture

The pre-B cell acute lymphoblastic cell line LK63 and LiBr melanoma lines were described previously (Boyd et al., 1992, Lawrenson et al., 2002) and cultured in RPMI, 10% FCS. Human kidney epithelial 293 (HEK293, ATCC) cells were maintained in DME, 10% FCS. Human Microvascular endothelial cells (HMVECs, Clonetics) were cultured in endothelial cell basal medium (EBM, Clonetics), supplemented with 5% FCS, Glutamine, Bovine Brain Extract (BBE), Hydrocortisone and GA-1000 (Gentamicin, Amphotericin B). In co-cultures, HMVECs were stimulated with LPS (1 µg/ml) or left untreated prior to addition of LK63 cells. Mouse cortical neurons were isolated from E 14.5 embryos and cultured in Neural Basal medium (Gibco) supplemented with appropriate growth factors.

Transfection of HEK 293 was carried out using Fugene 6 transfection reagent (Roche Biochemicals), and stable EphA3 (EphA3/HEK 293) and ephrinA5 (ephrin-A5/HEK 293) expressing cell clones were selected in 2µg/ml puromycin or 400µg/ml G418, by flow cytometry using anti EphA3 MAb or EphA3-Fc protein for detection, respectively.

### Alexa Fluor™ 546 conjugates and Dynalbeads

Recombinant, purified ephrinA5-Fc, EphA3-Fc, EphA1-Fc, and the recombinant human Fc protein were labelled using a (Alexa Fluor ™ 546 fluorescent labelling kit (Molecular Probes). Coupling of the ALEXA dye and its effect on the biological integrity of ephrin and Eph proteins were monitored by spectral (HPLC diode array detection) and BIAcore binding analysis during the labelling reaction. Specific binding of the labelled protein to sensor chip-coupled EphA3 extracellular domain or to ephrinA5 (ephrin-A1) respectively (Lackmann et al., 1997, Lackmann et al., 1998) was used to indicate biological integrity. Labelling reactions were terminated immediately when the first decrease in binding was detected.

EphrinA5-Fc Alexa Fluor™ 546 conjugate (ALEXA ephrinA5-Fc) or a non-relevant, ALEXA-labelled control protein were immobilised onto protein A-coated Dynabeads (Dynalbiotech) according to the manufacturer's instructions.

### Confocal microscopy and immunocytochemistry

Eph/ephrin stimulation was analysed in-situ by time-lapse confocal microscopy and immunocytochemistry as described (Lawrenson et al., 2002). To monitor internalisation of EphA3/ephrinA5 complexes and ephrinA5 shedding from the Dynabeads, cells plated on coverslips were stimulated with ALEXA ephrinA5-Fc or Alexa-labelled control proteins, either non-clustered or pre-clustered or coupled to Dynabeads. To visualise ephrinA5 internalisation into the lysosomal compartment, EphA3/HEK293 cells were incubated with Lysotracker™ green. Excess Lysotracker™ dye was removed by washing with media and replaced with pre-clustered Alexa ephrinA5-Fc. During the time-course, images were collected sequentially at two excitation wavelengths to minimise spectral overlap between different channels. The resulting green (Lysotracker™) and red (Alexa Fluor™546 ) signals were separated with a dichroic mirror and further filtered with barrier filters placed in front of separate detectors.

### Cell fractionation:

Cell fractionation and isolation of plasmamembranes was achieved using a cationic colloidal silica method as described (Stolz et al., 1992). Briefly, ephrin-stimulated, adherent cells were harvested into 10 mM EDTA/PBS, pooled with detached cells in the medium, washed with ice-cold PBS and re-suspended in 6% (w/w) cationic colloidal silica in coating buffer (20 mM MES, 150 mM NaCl, 280 mM sorbitol) to final concentration of 3% silica. The silica-coated cells were recovered by centrifugation (900g) and treated with 1 mg/ml poly-acrylic acid (Sigma) to block residual charges on the silica. Washed cells were suspended in lysis buffer (20 mM Tris-HCl, pH 7.5 containing protease inhibitors (Complete™, Boehringer), ruptured at 4°C by nitrogen cavitation (1200 p.s.i., Parr bomb) and silica-coated plasma membranes and cell nuclei collected by centrifugation.

Internal membranes contained in the supernatant were separated from the cytosolic fraction by centrifugation at 100,000g for 30 min. The plasma membrane pellet was layered onto a 60% Opti-prep (AXIS-Shield, Oslo, Norway) cushion in 20 mM Tris, pH 7.5 and the purified, silica-coated membranes collected as pellet after centrifugation (SW 60 Ti rotor) at 28,000g for 20 min. The membrane pellet was dissolved in 0.5% SDS and EphA3 was immunoprecipitated from this plasma membrane protein preparation using the á-EphA3 MAb IIIA4, and analysed together with total proteins contained in the other cell fractions by Western Blot analysis.

### Adhesion assays:

Ephrin coated surfaces were prepared as described previously (Lawrenson et al., 2002). Serum starved (4h) LK63 (2x10⁵cells/ well) and LiBr cells (5x10⁴cells/ well) were seeded onto wells coated with ephrinA5-Fc at indicated densities. Soluble, monomeric ephrinA5 was added as inhibitor (+ inhibitor) to parallel LK63 and LiBr cultures at 100-fold molar excess before seeding. After 4 hours, and extensive wash protocols (PBS) adherent cells were incubated with XTT reagent (Roche) at 37°C and quantitated after 4-12h by measuring the A₄₉₂ absorbance. The fraction of adherent LK63 cells was estimated by using values in wells lacking ephrin and containing most adherent cells as reference points, and correcting for A₄₉₂ absorbance in wells lacking LK63 cells. Data are expressed as mean +/- standard error (S.E.) and are representative of three independent experiments.

### Immunoprecipitation and Western blotting

Serum-starved (4 h, 1% FCS) cells were incubated with pre-clustered ephrinA5-Fc (1.5 i g), and at indicated times lysed in 50 mM Tris, pH 7.4, 150 mM NaCl, 1% Triton X100, 1 mM NaV0₄, 10 mM NaF and protease inhibitors (TBS-Tx100). Lysates were immunoprecipitated with IIIA4. Mab (Boyd et al., 1992) coupled to Mini Leak agarose beads (Kem-En-Tec A/S, Denmark), o/n at 4°C. Washed (TBS-Tx100) immunoprecipitates were analysed by Western Blot analysis with appropriate antibodies. Blots were visualised using an ECL substrate (Pierce).

### FRET microscopy

GFP EphA3 (w/t or mutant) expressing cells were stimulated, fixed, permeabilised and stained with Cy3-conjugated anti phosphotyrosine monoclonal antibody PY72 prior to mounting onto glass slides using Mowiol (Calbiochem). Fluorescence lifetime imaging microscopy (FLIM) sequences were obtained at 80 MHz with an Olympus IX70 microscope (100 /1.4 NA oil immersion lens) and analysed as described (Reynolds, Tischer et al. 2003). A 476-nm argon laser line and narrow-band emission filter (HQ510/20; Chroma) were used for GFP, a 100-W mercury arc lamp with high Q Cy3 filter set (excite, HQ545/30; dicroic, Q580LP; emitter, HQ610/75) for Cy3 and Alexa 546. GFP Fluorescence was detected with a dichroic beamsplitter (Q495 LP; Chroma Technology, Brattleboro, VT) and narrow-band emission filter. Stimulated FRET was measured in live 293 cells between transiently expressed ephrin-A2GFP and Alexa546 EphA3-Fc.

### Results

### The same EphA receptor elicits opposite responses in different tumour cells

To assess cell-morphological consequences of adherent and non-adherent, EphA3-positive cells to ephrinA5-exposure we examined LiBr melanoma cells or LK63 pre-B leukemia cells cultured on fibronectin or ephrinA5-Fc coated surfaces by confocal microscopy. On fibronectin, LiBr melanoma cells are firmly attached and spread, revealing distinct dendritic cell processes (Lawrenson et al., 2002) and rhodamine phalloidin-stained actin stress fibres (Figure 1A). By contrast, the majority of LK63 cells are suspended and the few cells trapped on the slide exhibit a cortical actin cytoskeleton at the cell periphery (Figure 1A, top). Exposure to surface-anchored ephrinA5-Fc dramatically affects both cell types, but in opposite directions: LK63 leukemia cells develop a flat, irregular shape and their distinct cortical actin ring converts into a diffuse actin cytoskeleton. Moreover, they extend conspicuous, filopopodia-like actin rich protrusions (arrowheads) that seem to tether the cells onto the ephrin-containing substratum (Figure 1A, bottom). By contrast, the majority of LiBr melanoma cells fail to attach to the ephrinA5-coated surface, whereby the remaining cells mostly are contracted, round and have little or no contact to the substratum. These changes in melanoma cell morphology are accompanied by redistribution of polymerised actin into dense cortical actin rings (figure 1A, bottom, right, (Lawrenson et al., 2002).

Surfaces of protein-A coated cell culture plates conjugated with defined densities of ephrinA5-Fc allowed to examine the dose-dependence of the opposing adhesive and repulsive cell responses. With mounting ephrin5 surface density LK63 cells become increasingly adherent (Figure 1 B, LK63), and maximal cell adhesion is apparent at 1.0 - 1.8 ng ephrinA5-Fc/mm². LiBr melanoma cells moderately adhere in the absence of ephrinA5, and at very low ephlin density. Increasing ephrinA5 surface concentration results in their dose-dependent cell detachment from the culture well (Figure 1B, LiBr). Importantly, competing, soluble ephrin-A5 effectively abrogates LK63 attachment and LiBr repulsion, and demonstrates specificity of the cell responses for the ephrinA5/EphA3 interaction (Figure 1B, LiBr + inh, LK63 + inh.). We assessed if ephrin-A5 induced repulsion of EphA3-positive melanoma cells also results in increased propensity of these cells to invade through three-dimensional collagen gels away from the ephrin source towards a chemoattractant (3T3-conditioned media). While the presence of ephrin in the bottom chamber did not change the invasive capacity of the melanoma cells in the upper chamber, the addition of clustered ephrin-A5 Fc together with the cells doubled the number of invasive cells migrating through the membrane (Figure 1C, top panel). We confirmed this response with ephrin-A5 expressing 293 cells. LiBr cells that had been stained with fluorescent Cell Tracker dye were placed together with ephrin-A5/HEK 293 cells in the top chamber and invasive cells on the underside of the membrane counted under the fluorescence microscope. As expected, the presence of ephrin-expressing cells increased the invasive capacity of the melanoma cells several-fold, an effect that was abrogated by the addition of non-clustered, soluble ephrin-A5 as competitive inhibitor in the top chamber (Figure 1C, bottom).

To examine EphA3-mediated cell adhesion in a different setting, we added LK63 cells to monolayers of ephrin5 positive E14.5 mouse cortical neurons (Figure 2A, I-IV) or ephrinA5/293 cells (Figure 2A, V-VII, Fig.2B), grown on coverslips. Immuno-cytochemical (Figure 2A, I, II, V, VI) and confocal time-lapse analysis (Fig. 2B) indicate that LK63 cells avidly attach to both, ephrin-A5/293 cells and mouse cortical neurons. Notably, this adhesion is accompanied by pronounced spreading of LK3 cells and development of actin-rich, filopodia-like extensions (Figure 2A, II, IV, arrowheads). LK63 adhesion is abrogated by blocking endogenous EphA3 with excess soluble ephrinA5 prior to plating (Figure 2A, III and VII), and parental HEK293 cells do not facilitate notable LK63 attachment or morphology changes (Figure 2A, VIII), suggesting that cell attachment is mediated through the Eph/ephrin interaction. Reversible labelling of cell surface ephrin-A5 with limiting amounts of Alexa EphA3-Fc (figure 2B) enabled us to monitor binding of LK63 cells to an ephrinA5/HEK 293 monolayer in real time, and to record dynamic morphological responses in ephrinA5-harbouring cells and in EphA3-positive LK63 cells simultaneously (Figure 2B). Rapid accumulation of LK63 cells on the ephrinA5/293 cell monolayer within 11min of addition suggests that direct contact between the two cell types leads to immediate cell-cell adhesion (Figure 2B).

This attachment is persistent and in prolonged co-cultures of these cells leads to large clusters of LK63 cells that remain tethered to the ephrinA5/293 cell monolayer. While shortly after initial contact pronounced membrane blebbing is apparent in LK63 cells (Figure 2B, 1', ), ephrinA5/HEK 293 cells extend Alexa EphA3-Fc stained (*i.e*., ephrinA5-rich), filopodia-like extensions that appear to mediate attachment of LK63 cells (Figure 2B, arrows). LK63 cell binding is accompanied by rounding and retraction of the ephrinA5/HEK 293 cells, resulting in the loss of cell-cell contacts within the ephrinA5/293-monolayer after 21-31 min (Figure 2B, 21', 31', arrowheads). Furthermore, a marked redistribution of the Alexa-tag and dynamic formation of distinct fluorescent clusters during LK63 cell attachment (Figure 2B) suggests that exposure to EphA3-expressing cell surfaces triggers notable redistribution of ephrin-A5 within the ephrinA5/HEK 293-cell membrane.

### EphA3/ephrin-A5 facilitated cell-cell adhesion does not rely on integrin ligation.

We examined the involvement of lymphocyte cell adhesion molecules by using function-blocking antibodies to abrogate LK63 adhesion to either ephrin-A5/HEK 293 cells or to LPS-stimulated HMVECs. Vascular and intercellular cell adhesion molecule (VCAM-1 and ICAM-1) are essential for adhesion and migration of normal lymphocytes (Bevilacqua, 1993) and of leukemia cells (Vincent et al., 1996), and immuno-cytochemical analysis confirms their expression on LK63 cells (Figure 3A, I, V). In control experiments, LK63 cells adhere notably to HMVECs and undergo characteristic cell morphological changes only after stimulation with LPS (Figure 3A, IX - XII, 3B). As expected, treatment with neutralising á-ICAM and á-VCAM antibodies effectively abrogates this LPS-induced LK63 adhesion (Figure 3A, VI, VII, 3B), while addition of excess soluble ephrinA5 has no effect (not shown). By contrast, EphA3/ephrin mediated LK63 attachment to ephrin-A5/293 cells is not impaired by á-VCAM-1 and à-ICAM-1 antibodies, added individually or in combination (Figure 3A, II, III, VI, VII, 3B), and confirms that this cell-cell adhesion solely relies on the tether provided by the Eph/ephrin interaction.

### Eph/ephrin mediated cell-cell repulsion, but not adhesion, coincides with ephrin-A5 internalisation.

The finding that EphA3 can facilitate either cell-cell adhesion or repulsion prompted us to analyse underlying cell-biological and biochemical pathways. Ligand-induced Eph receptor internalisation has not been described to date, but it is tempting to speculate that mechanisms regulating other RTKs may also play a role in the cellular responses observed in our experiments. To monitor the localisation of ligand during cell stimulation we prepared a fluorescent Alexa546-conjugate of ephrinA5-Fc (Alexa ephrinA5-Fc). Confocal time-lapse analysis reveals that within minutes of addition a fluorescent signal is evenly distributed around the EphA3/293 cell membranes (Figure 4A). After 5-10 minutes, distinct patches of fluorescence around the cell perimeter suggest ligand/receptor clustering (arrowheads), followed by formation of small fluorescent vesicles that detach from the membrane and appear in the cytosol (video, Figure 4A). Most of the fluorescent signal has disappeared from the membranes within 120 min of stimulation and has accumulated into large, cytosolic clusters (not shown). By contrast, LK63 cells that had been exposed to clustered Alexa ephrinA5-Fc in the same manner exhibited strong plasma membrane staining but did not reveal any signs of receptor/ligand internalisation (Figure 4B). We sought to confirm that internalisation relies on pre-clustering of ephrinA5-Fc, known to trigger EphA3 activation. Addition of non-clustered Alexa ephrinA5-Fc to EphA3/293 cells yielded distinct fluorescent staining of the plasma membrane, but little evidence for the formation of fluorescent cytosolic vesicles (Figure 4 C, III). Furthermore, Alexa ephrinA5-Fc stimulation in the presence of excess human IgG ("Fc block") to block potential Fc-receptor mediated uptake, did not abrogate ephrinA5-Fc internalisation (Figure 4 C, II), confirming an EphA3-specific, Fc-receptor independent mechanism.

We characterised the internalisation of Alexa ephrinA5-Fc by monitoring the lysosomal compartments of stimulated EphA3/293 cells during a confocal time-lapse experiment using Lysotracker™ green (Figure 4D). Green cytosolic vesicles prior to stimulation mark the lysosomal compartment (Figure 4D, 0 min). Following addition of Alexa ephrinA5-Fc (red), its rapid binding (Figure 4D, 5min), clustering (5, 35 min) and internalisation (35, 90 min) are apparent. The merged red (Alexa ephrinA5-Fc) and green (Lysotracker™) images indicate internalisation of ephrinA5 and its co-localisation to the lysosomes.

### EphA3/epphrinA5 facilitated cell-cell adhesion or repulsion involves distinct molecular pathways

We sought to examine molecular pathways involved in EphA3-mediated cell repulsion or adhesion, by IP/Western Blot analysis of whole-cell lysates or isolated cell-compartments from ephrin5 stimulated, EphA3 expressing cells. Most strikingly, EphA3 receptors in EphA3/293 and LK63 leukemia cells differ profoundly in the level of their EphA3 tyrosine phosphorylation (Fig. 5A, bottom panel). As demonstrated previously (Lawrenson et al., 2002), EphA3/HEK 293 cells respond to stimulation with clustered ephrin5 by rapid increase in EphA3 phosphorylation within 10 min. By contrast, in LK63 cells EphA3 is phosphorylated only marginally after 60min stimulation Interestingly, in LK63 cells but only very weakly in EphA3/293 cells, ephrinA5-Fc-binding to EphA3 is accompanied by rapid recruitment of the protein tyrosine phosphatase SHP-2, suggesting its activity might be involved in maintaining the low level of tyrosine phosphorylated EphA3 in LK63 cells (Fig. 5A, middle panel).

Our finding that EphA3 facilitated cell repulsion coincides with internalisation of the receptor/ligand complex (Figure 4) prompted us to assess the involvement of the adaptor protein c-Cbl in EphA3 signalling. Cbl acts downstream of growth factor and cytokine receptors and integrins and is known to effect their down-modulation, ubiquitination and endocytic degradation (Andoniou et al., 1996, Thien and Langdon, 2001). Constitutive association of c-Cbl with EphA3 was apparent in whole cell lysates of LK63 cells, EphA3/293 cells (Fig. 5A) and A09 melanoma cells (not shown). However, while remaining EphA3-bound in EphA3/293 cells, c-Cbl dissociates from the receptor in LK63 cells upon stimulation (Fig. 5A). Immunoprecipitation of tyrosine-phosphorylated proteins from EphA3/293 cells revealed rapid phosphorylation of endogenous c-Cbl within 1 min of ephrinA5 stimulation (Fig. 5B).

We sought to examine the fate of internalised EphA3/ephrinA5 complexes in individual cell compartments. Fractionation of hypotonic cell lysates from colloidal silica-coated cells by density gradient centrifugation allows effective separation between plasma membrane, cytosol and internal membranes (Stolz et al., 1992). Western blot analysis of ephrin stimulated, EphA3/293 cells revealed increasing ubiquitination of a protein corresponding to the apparent size of EphA3 in samples of whole-cell lysates and isolated plasma membrane fractions (Figure 5C) of EphA3/293 cells within 15 minutes of stimulation. By contrast to the EphA3/293 cells, ephrinA5-treated LK63 cells revealed a complete lack of EphA3 ubiquitination (Fig. 5D), in agreement with the observation that c-Cbl rapidly dissociates from the ephrin-tethered receptor (Fig. 5A).

### EphrinA5 binding to EphA3/293 but not to LK63 cells is followed by its rapid cleavage and internalisation.

To assess if internalisation requires cleavage of surface-tethered ephrinA5, we conjugated Alexa ephrinA5-Fc onto Protein-A Dynabeads, simulating a high-density ligand surface for incubation with EphA3 positive and control cells. Both, LK63 and EphA3/HEK 293 cells rapidly bound the Alexa ephrinA5-Fc-labelled beads (Figure 6A, I, III). In the case of EphA3/HEK 293 cells (I), the Alexa fluorescence dispersed locally from the bead surface and within minutes distributed over the cell membrane. Internalisation was obvious within 15 min and resulted in the formation of characteristic fluorescent vesicles in the cytosol (compare Figures 4A and 6A). In control experiments, parental HEK 293 cells (not shown) or EphA3/HEK 293 cells exposed to non-relevant Alexa EphA1-Fc Dynabeads (Figure 6A, II, EphA1 Bd) did not reveal any signs of cleavage or internalisation. Others have demonstrated recently that cleavage of the ephrin-A2/EphA3 interaction during axon repulsion is facilitated by ADAM10, a metalloprotease that is also present on HEK293 cells (Hattori et al., 2000). Incubation of EphA3/HEK 293 cells with the metalloprotease inhibitor 1', 10'-O-Phenanthroline prior to addition of Alexa ephrin-A5-Fc beads completely abrogated ephrin cleavage and internalisation (Fig. 6B, panel II), suggesting the likely involvement of ADAM10 or a related protease in this reaction.

In contrast to the response of EphA3/293 cells, there was no evidence for cleavage or internalisation of Alexa ephrinA5 into LK63 cells (Fig. 6A, III). However, the ephrinA5 loaded beads remained tightly cell-bound during the experiments (video Figure 6AIII) and provoked distinct membrane blebbing (Fig. 6A, III, arrowheads). Western blot analysis of immuno precipitates from LK63, LiBr melanoma or EphA3/293 cells indicated in agreement, association of ADAM10 with EphA3 in EphA3/293 and melanoma cells, but not in LK63 cells (Figure 6C). To demonstrate ephrin cleavage biochemically, EphA3/HEK 293 cells were treated with pre-clustered ephrin-A5 Fc. Following absorption of Fc-tethered ephrin with protein-A, cleaved ephrin was precipitated from pooled cell supernatants and Triton-X100-lysates of EphA3/293 cells with protein-A-bound EphA3-Fc and monitored by Western blot with anti- ephrinA5 antibody (Figure 6D). Cleaved ephrinA5 was observed in EphA3/HEK 293 cells treated with clustered, but not with non-clustered ephrin-A5 Fc, while the same treatment yielded significantly-reduced ephrin cleavage in parallel EphA3/HEK 293 cultures treated with 1',10'-o-phenanthroline to block cleavage by metalloproteases. Interestingly, 293T cells transfected with EphA3 containing an inactivating mutation within the kinase domain (K₆₅₃M EphA3) showed only marginal ephrin cleavage after stimulation with pre-clustered ephrin-A5 (Figure 6D) suggesting a role of EphA3 kinase activity in this process.

Together, these results indicate that in the absence of ephrinA5-cleavage and hindrance of receptor phosphorylation, the EphA3/ephrinA5-interaction switches from cell repulsion to Eph/ephrin-mediated cell adhesion.

### Phosphatase inhibitors trigger Eph receptor phosphorylation

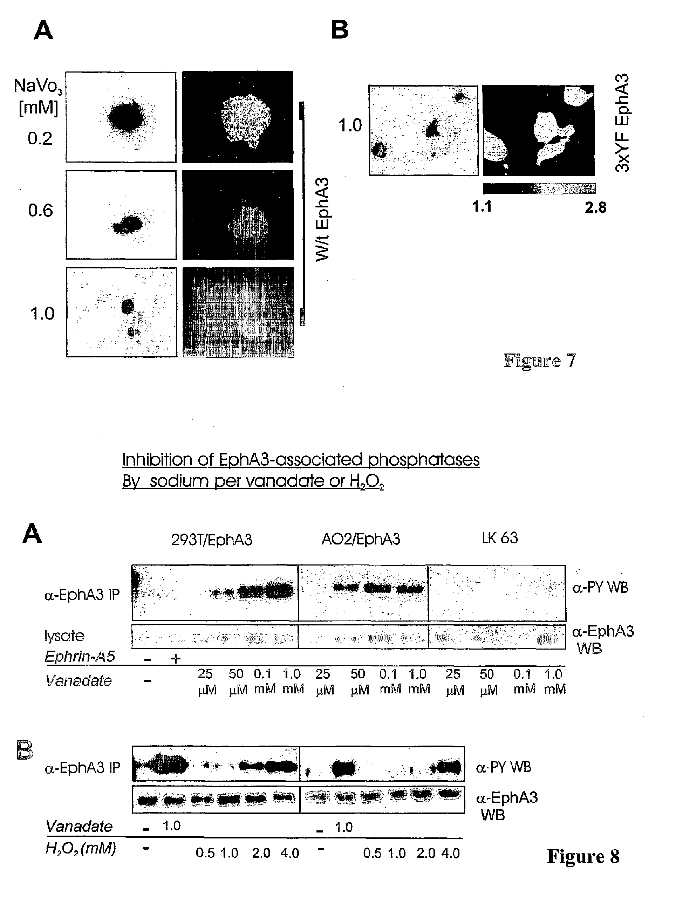

Global activation of EphA3 was monitored by Fluorescent Lifetime imaging microscopy (FLIM) after blocking cytosolic tyrosine phosphatase activity. Treatment of EphA3-GFP w/t expressing cells with the phosphatase inhibitor sodium-pervanadate leads to a dramatic dose-dependent decrease in the GFP fluorescent lifetime across the whole cell surface (Figure 7A), indicative of universal receptor phosphorylation. By contrast, cells expressing mutant EphA3GFP (3YF EphA3GFP), deficient of juxta-membrane and activation-loop tyrosines, (Lawrenson, Wimmer-Kleikamp et al. 2002), retained their GFP lifetime even after treatment with high concentrations of pervanadate (Figure 7B), confirming the specificity of the FRET analysis but also assigning these three tyrosines as principle, *in-vivo* EphA3 phosphorylation sites in w/t EphA3-expressing cells (Figure 7A).

### Dose dependent Eph receptor phosphorylation

The observation that EphA3 is only marginally phosphorylated in LK63 cells upon ephrin stimulation raises the important question as to how the phosphorylation level is down-regulated in those cells. In agreement with the FLIM data, treatment of EphA3 over-expressing (by stable transfection) EphA3/HEK 293 and EphA3/A02 malignant melanoma cells with increasing amounts of pervanadate or H₂O₂ leads to a dose dependent increase in EphA3 phosphorylation (Figure 8 A,B). However, at the highest vanadate concentrations tested (1 mM) EphA3 phosphorylation in LK63 cells is significantly lower despite comparable EphA3 levels, possibly suggesting increased tyrosine protein tyrosine phosphatase activities in these cells (Figure 8A, right panel). If indeed elevated phopshatase activity is responsible for the unusual response of these cells to cell surface ephrin-A5, then phosphatase inhibition should prevent LK63 adhesion to surface-tethered ephrin.

In agreement, LK63 cells, seeded onto ephrin- or FN coated glass coverslips as described in Figure 1A but treated with sodium-pervanadate (vanadate) lose their characteristic extensions and appear rounded, their actin cytoskeleton changing to condensed cortical actin rings, as seen in the ephrin-stimulated melanoma cells (Figure 9). In the absence of this vanadate treatment, LK63 cells adhere to ephrin coated surfaces, and exhibit a diffuse actin cytoskeleton and lamellipodia-like extensions directed towards the ephrin coated surface.

### LMW- PTP associates with EphA3 and influences receptor phosphorylation.

The obvious involvement of PTPs in EphA3 signalling led us to explore which phosphatase(s) are responsible for the observed effects. Immunoprecipitation analysis with antibodies against known phopshatases suggested association of both, SHP2 and LMW-PTP. The latter is a likely candidate as it has been shown to influence EphB2, EphB1 (Stein, Lane et al.) and EphA2 signaling (Kikawa, Vidale et al. 2002). In agreement, w/t LMW-PTP overexpressing EphA3/ HEK293 cells reveal a lack of EphA3 phosphorylation after ephrin-A5 stimulation (Figure 10B). By contrast, ephrin-A5 stimulation triggers pronounced EphA3 phosphorylation in EphA3/HEK 293 cells transfected with dominant negative LMWPTP or vector only, suggesting that this phosphatase indeed influences the EphA3 phosphorylation. In agreement, we detected association of endogenous LMWTP with EphA3 in lysates of EphA3/HEK 293 cells and malignant melanoma cells (AO2, AO9, Figure10 A).

### Overexpression of LMWPTP abrogates ephrin-A5 mediated cell rounding and actin cytoskeletal changes.

To assess the functional relevance of LMWTP association with EphA3 we monitored actin-cytoskeletal changes in EphA3-over expressing 293T cells which were transfected with either w/t or dominant-negative LMWPTP (d/n) constructs. In the absence of ephrin, all cells were extensively spread and revealed distinct actin-rich cell processes. Importantly and in agreement with the biochemical data (Figure11), treatment with pre-clustered ephrin-A5 Fc leads to rounding, and contraction of the actin cytoskeleton only in d/n LMWPTP-transfected and vector-transfected control cells. By contrast, w/t LMWPTP-overexpressing cells expressing cells do not to respond to ephrin-A5 treatment and do not change their morphology during the experiment, indicating functional involvement of this phosphatase activity in EphA3 signalling.

### EphA3/ephrinA5 facilitated diametrically opposed responses are influenced by Eph kinase activity and phosphorylation

To examine whether EphA3 tyrosine phophorylation and kinase activity influence the molecular switch between Eph/ephrin mediated repulsion and adhesion, EphA3 negative AO2 malignant melanoma cells stably expressing EphA3 w/t or signalling compromised EphA3 mutants harbouring mutations in the three major phosphorylation sites (3YF EphA3) or lacking the entire cytoplasmic domain (EphA3Δcyto) were challenged with clustered ephrin-A5 Fc while parallel cultures were left unstimulated. Since endogenous EphA3 expression in A02 cells is low to undetectable by Northern Blot and FACS analyis, these cells are ideally suited for stable transfection of EphA3 constructs. Fixed and Alexa 488 phalloidin stained cells in the absence of ephrin possess adherent cell bodies with extensive processes and actin stress fibres (Figure 12 A, left panel). In analogy to the cellular repulsion response observed in Libr melanoma cultures (Lawrenson, Wimmer-Kleikamp et al. 2002), stimulation of EphA3/A02 cells with clustered ephrin is accompanied by cell rounding, redistribution of polymerised actin into dense cortical actin rings (Figure 12A, w/t) and cell repulsion. Adhesion assays under the same experimental conditions confirmed these findings and only about 6% of the starting LiBr and 45% of the EphA3/ A02 population remained attached to the tissue culture surface when exposed to ephrin (adhesion assay, Figure 12B). Parallel parental AO2 control cultures, by comparison, showed no change in morphology and most cells remained attached throughout the experiment (Figure 12A, B, control). Importantly, expression of C- terminal mutated, kinase-inactive (EphA3Δcyto) or tyrosine mutated EphA3 (3YF EphA3) abrogated the Eph/ephrin mediated repulsion response and cells remained spread out and adherent with a similar morphology and actin cytoskeleton to non-ephrin stimulated cells (Figure 12 A, B).

### Discussion

EphA3 was first isolated as cell surface antigen from LK63 lymphoblastic pre-B cells (Boyd et al., 1992) and independently identified in malignant melanoma cells (Chiari et al., 2000), which respond to ephrinA5 stimulation with contraction of the cytoskeleton and cell detachment (Lawrenson et al., 2002). We now demonstrate that in LK63 cells ephrinA5 exposure has the opposite effect and facilitates EphA3-mediated cell attachment. We show the concurrent loss of a distinct cortical actin ring, typical for non-stimulated LK63 cells, and gain of a diffuse actin network, cell spreading and the development of actin-rich, filopodia-like extensions, which appear to tether the cells to the ephrinA5 surface. EphrinA5 induced repulsion is accompanied by rapid cleavage of surface-tethered ephrin-A5, internalisation and lysosomal degradation. By contrast, ephrin cleavage and internalisation are not apparent in LK63 cells, which bind integrin-independent to surface-tethered ephrin-A5.

Firstly, attachment of non-adherent LK63 pre-B leukemia cells to ephrin-A5-decorated tissue culture surfaces, cortical neurons or ephrin-A5/293 cells, but not to LPS-stimulated HMVECs, is inhibited with excess soluble ephrinA5, demonstrating requirement for ephrin-A5/EphA3 mediated cell-cell contacts. While we have not detected ephrinA5 protein expression in HMVECs, we recently confirmed ephrin-A5/EphA3-dependent LK63 adhesion to ephrinA5 expressing primary human endothelial tumour cells.

Secondly, blocking of integrin receptors on LK63 cells with neutralising anti- ICAM-1 and anti- VCAM-1 antibodies does not affect LK63 adhesion to ephrin-A5/293 cells, while adhesion to LPS-stimulated HMVECs is significantly reduced. In accordance with their immune surveillance function lymphocytes and leukemia cells respond to LPS stimulation by rapid adhesion to the vascular endothelium. This response is mediated predominantly by the ICAM-1 and VCAM-1 integrin receptors (Bevilacqua, 1993), both of which are expressed abundantly on LK63 cells.

However, the cell morphological changes accompanying LPS-induced adhesion of LK63 cells to HMVECs, including cell spreading and development of an extensive actin cytoskeletal network are very similar to those observed during exposure to cell surface-tetherered ephrinA5 (compare Figures 1A, 3A). Our observation supports the recently proposed notion that Eph receptor-mediated cell-cell tethering and accompanying cytoskeletal changes may not follow 'classical integrin-mediated' mechanisms (Carter et al., 2002).

Thirdly, the conceptual requirement for Eph/ephrin-mediated repulsion of releasing the interacting cells by cleavage of the molecular (Eph/ephrin) link, infers that lack of cleavage could promote cell-cell attachment. Indeed, an important study demonstrated recently that abrogation of EphA3 dependent, ADAM -catalysed ephrin-A2 cleavage at sites of cell-cell contact prevents axon repulsion (Hattori et al., 2000). In a number of analysed ephrinA2 expressing cells, including HEK293 and NIH3T3 cells, binding of clustered EphA3-Fc was shown to trigger proteolytic cleavage of membrane-bound ephrinA2.

Intriguingly, our experiments, examining cleavage of ephrinA5-Fc immobilised to Protein-A beads, suggest that EphA3 expressing cells can induce ephrin cleavage in *trans.* The apparent activation of this metalloprotase activity by surface-tethered ephrinA5 binding to EphA3/293 cells, but not to LK63 cells, raises important questions about its identity and activation mechanism. Immunoprecipitation analysis indicates that a Kuzbanian-type protease, possibly ADAM10, associates with EphA3 in EphA3/293 but not in LK63 cells. Interestingly, constitutive association of ADAM10 with ephrinA2 seems to involve an ephrin motif (Hattori et al., 2000) that aligns with the high-affinity receptor binding loop of the corresponding ephrin-B2 structure (Himanen et al., 2001). This configuration suggests that ADAM-10 displacement during the Eph/ephrin interaction could act as trigger to activate ephrin cleavage. In agreement, in our experiments the release and internalisation of tethered ephrinA5 are abrogated in the presence of the protease inhibitor 1,10-O-Phenanthroline. Importantly, while ephrinA5-Fc coated beads bind avidly to LK63 cells, we do not find any signs of ligand cleavage or internalisation, indicating that deficiency in EphA3-associated metalloprotease activity is responsible for persisting EphA3/ephrinA5 mediated adhesion.

While mechanisms that terminate ephrin-induced Eph signals and are involved in Eph receptor trafficking have not been described to date, the fast kinetics of ephrin-induced cytoskeletal responses observed in many studies (Zou et al., 1999, Miao et al., 2000, Elowe et al., 2001, Lawrenson et al., 2002, Carter et al., 2002) suggest a rapid turn-over of Eph/ephrin complexes. We have addressed this aspect of Eph function for the first time and observe that cell repulsion of EphA3/293 cells, but not LK63 cell adhesion is accompanied by rapid internalisation of the receptor/ligand complexes and their accumulation in the lysosomal compartment. Concurrent phosphorylation of EphA3-associated c-Cbl, which facilitates poly-ubiquitination and degradation of many activated RTKs (Thien and Langdon, 2001), as well as prominent EphA3 ubiquitination, suggests Eph degradation as important component of ephrin-induced cell repulsion. By contrast, the apparent lack of ubiquitination in LK63 cells, and dissocation of c-Cbl from ephrinA5 bound EphA3 coincide with EphA3/ephin-A5 cell surface complexes persisting as stable molecular tether between interacting cells. In support of our observations, in Jurkat and COS-7 cells c-Cbl is constitutively associated with EphB6, an Eph receptor lacking intrinsic kinase activity, and ephrin binding causes c-Cbl dephosphorylation and dissociation from SHP1 (Luo et al., 2001, Freywald et al., 2002).

It is noteworthy, that internalisation is dramatically reduced when non-clustered, ephrinA5-Fc was applied to EphA3/293 cells, suggesting that signalling from ligand-activated, phosphorylated EphA3 is required for efficient endocytosis of EphA3/ephrinA5 complexes. Intriguingly, our data demonstrate that in EphA3/HEK 293 and LK63 leukemia cells EphA3 receptors differ profoundly in the level of their tyrosine phosphorylation (Fig. 3A, bottom panel). Whereas EphA3/293 cells respond to ephrinA5 stimulation by rapidly increasing EphA3 phosphorylation (Lawrenson et al., 2002), only marginally phosphorylated EphA3 is found in ephrin-treated LK63 cells. Importantly, our results also reveal constitutive association of the protein tyrosine phosphatase (PTP) LMW-PTP, and in LK63 but not in EphA3/293 cells, that ephrinA5 binding to EphA3 is accompanied by rapid recruitment of the PTP SHP-2 (Fig. 3A, middle panel). Overexpression of w/t LMW-PTP dramatically decreases EphA3 phosphorylation after ephrin-A5 stimulation, while overexpression of the dominant-negative (d/n) LMW-PTP mutant increases the phosphorylation level after ephrin-A5 stimulation, suggesting that LMW-PTP is involved in regulating EphA3 phosphorylation levels and thereby also regulating the activity of the kinase function. In agreement, overexpression of d/n LMW-PTP, presumably blocking the inhibitory affect of associated endogenous LMW-PTP and resulting in activation of the EphA3 receptor, induces cell rounding. Furthermore, it seems possible that SHP-2 is involved in maintaining the low level of tyrosine phosphorylated EphA3 in LK63 cells, which in turn affect EphA3 signalling and the resulting cytoskeletal response. SHP-2 is a ubiquitously expressed PTP known to regulate cell adhesion, spreading, migration or integrin induced chemotaxis by modulating tyrosine phosphorylation of focal adhesion components (Oh et al., 1999, Manes et al., 1999, Saxton and Pawson, 1999). Its involvement in Eph signalling is suggested from the finding that EphA2 and EphB2-mediated cell rounding and de-adhesion of PC3 prostate epithelial cells and transfected 293 cells involves SHP-2 recruitment and dephosphorylation of focal adhesion kinase (Miao et al., 2000, Zou et al., 1999).

Eph/ephrin mediated cell-cell communication is essential for the establishment of tissue patterns during embryogenesis (Holder and Klein, 1999, Mellitzer et al., 1999) and phenotypes of Eph or ephrin mutant invertebrates and mice have emphasised the importance of Eph kinase-dependent and independent cell repulsion and adhesion mechanisms (Boyd and Lackmann, 2001). Our findings presented here demonstrate that in EphA3-positive tumour cells notable differences in EphA3 kinase activity and downstream signalling components, as well as in cell-associated metalloprotease activity, determine the net cell-biological response to ephrinA5 exposure, propagating either Eph kinase-dependent cell contraction and detachment or kinase-independent, Eph-ephrin facilitated cell attachment and spreading. Furthermore, they support the intriguing possibility that Eph/ephrin associated metalloproteases function as trigger to activate the molecular switch between Eph/ephrin-mediated cell-cell adhesion and cell repulsion.

Throughout the specification the aim has been to describe the preferred embodiments of the invention without limiting the invention to any one embodiment or specific collection of features. It will therefore be appreciated by those of skill in the art that, in light of the instant disclosure, various modifications and changes can be made in the particular embodiments exemplified without departing from the scope of the present invention.

### REFERENCES

Adams,R.H., G.A.Wilkinson, C.Weiss, F.Diella, N.W.Gale, U.Deutsch, W.Risau, and R.Klein. 1999. Roles of ephrinB ligands and EphB receptors in cardiovascular development: demarcation of arterial/venous domains, vascular morphogenesis, and sprouting angiogenesis. Genes Dev 13:295-306.
Andoniou,C.E., C.B.Thien, and W.Y.Langdon. 1996. The two major sites of cbl tyrosine phosphorylation in abl-transformed cells select the crkL SH2 domain. Oncogene 12:1981-1989.
Batlle,E., Henderson J.T., H.Beghtel, G.Huls, M.van de Wetering, T.Pawson, and H.Clevers. 2002. b-Catenin and TCF Mediate Cell Positioning in the Intestinal Epithelium by Controlling the Expression of EphB/EphrinB. Cell 111:251-263.
Becker,E., U.Huynh-Do, S.Holland, T.Pawson, T.O.Daniel, and E.Y.Skolnik. 2000. Nck-interacting Ste20 kinase couples Eph receptors to c-Jun N-terminal kinase and integrin activation. Mol Cell Biol. 20:1537-1545.
Bevilacqua,M.P. 1993. Endothelial-leukocyte adhesion molecules. Annu. Rev. Immunol. 11:767-804.
Birgbauer,E., C.A.Cowan, D.W.Sretavan, and M.Henkemeyer. 2000. Kinase independent function of EphB receptors in retinal axon pathfinding to the optic disc from dorsal but not ventral retina. Development 127:1231-1241.
Birgbauer,E., S.F.Oster, C.G.Severin, and D.W.Sretavan. 2001. Retinal axon growth cones respond to EphB extracellular domains as inhibitory axon guidance cues. Development. 128:3041-3048.
Boyd,A.W. and M.Lackmann. 2001. Signals from Eph and ephrin proteins: a developmental tool kit. Sci STKE. 2001:RE20.
Boyd,A.W., L.D.Ward, I.P.Wicks, R.J.Simpson, E.Salvaris, A.Wilks, K.Welch, M.Loudovaris, S.Rockman, and I.Busmanis. 1992b. Isolation and characterization of a novel receptor-type protein tyrosine kinase (hek) from a human pre-B cell line. J. Biol. Chem. 267:3262-7.
Carter,N., T.Nakamoto, H.Hirai, and T.Hunter. 2002. EphrinA1-induced cytoskeletal re-organization requires FAK and p130(cas). Nat Cell Biol 4:565-573.
Chiari,R., G.Hames, V.Stroobant, C.Texier, B.Maillere, T.Boon, and P.G.Coulie. 2000. Identification of a tumour-specific shared antigen derived from an Eph receptor and presented to CD4 T cells on HLA class II molecules. Cancer Res. 60:4855-4863.
Coulthard,M.G., J.D.Lickliter, N.Subanesan, K.Chen, G.C.Webb, A.J.Lowry, S.Koblar, C.D.Bottema, and A.W.Boyd. 2001. Characterization of the Epha1 receptor tyrosine kinase: expression in epithelial tissues. Growth Factors 18:303-317.
Dodelet,V.C. and E.B.Pasquale. 2000. Eph receptors and ephrin ligands: embryogenesis to tumourigenesis. Oncogene 19:5614-5619.
Elowe,S., S.J.Holland, S.Kulkarni, and T.Pawson. 2001. Downregulation of the Ras-mitogen-activated protein kinase pathway by the EphB2 receptor tyrosine kinase is required for ephrin-induced neurite retraction. Mol. Cell. Biol. 21:7429-7441.
Freywald, A., N.Sharfe, and C.M.Roifman. 2002. The Kinase-null EphB6 Receptor Undergoes Transphosphorylation in a Complex with EphB1. J Biol Chem 277:3823-3828.
George,S.E., K.Simokat, J.Hardin, and A.D.Chisholm. 1998. The VAB-1 Eph receptor tyrosine kinase functions in neural and epithelial morphogenesis in C. elegans. Cell 92:633-643.
Gu,C. and S.Park. 2001. The EphA8 receptor regulates integrin activity through p110gamma phosphatidylinositol-3 kinase in a tyrosine kinase activity-independent manner. Mol Cell Biol 21:4579-4597.
Hamann,P.R. et al. 2002. Bioconjugate Chem. 13, 47-58.
Hattori,M., M.Osterfield, and J.G.Flanagan. 2000. Regulated cleavage of a contact-mediated axon repellent. Science 289:1360-1365.
Himanen,J.P., K.R.Rajashankar, M.Lackmann, C.A.Cowan, M.Henkemeyer, and D.B.Nikolov. 2001. Crystal structure of an Eph receptor-ephrin complex. Nature 414:933-938.
Holder,N. and R.Klein. 1999. Eph receptors and ephrins: effectors of morphogenesis. Development 126:2033-2044.
Holmberg,J., D.L.Clarke, and J.Frisen. 2000. Regulation of repulsion versus adhesion by different splice forms of an Eph receptor. Nature 408:203-206.
Holmberg,J. and J.Frisen. 2002. Ephrins are not only unattractive. Trends. Neurosci. 25:239-243.
Huynh-Do,U., E.Stein, A.A.Lane, H.Liu, D.P.Cerretti, and T.O.Daniel. 1999. Surface densities of ephrin-B1 determine EphB1-coupled activation of cell attachment through alphavbeta3 and alpha5betal integrins. EMBO J 18:2165-2173.
Kullander,K., N.K.Mather, F.Diella, M.Dottori, A.W.Boyd, and R.Klein. 2001. Kinase-dependent and kinase-independent functions of EphA4 receptors in major axon tract formation in vivo. Neuron 29:73-84.
Lackmann,M., R.J.Mann, L.Kravets, F.M.Smith, T.A.Bucci, K.F.Maxwell, G.J.Howlett, J.E.Olsson, T.Vanden Bos, D.P.Cerretti, and A.W.Boyd. 1997. Ligand for EPH-related kinase (LERK) 7 is the preferred high affinity ligand for the HEK receptor. J Biol. Chem. 272:16521-16530.
Lackmann,M., A.C.Oates, M.Dottori, F.M.Smith, C.Do, M.Power, L.Kravets, and A.W.Boyd. 1998. Distinct subdomains of the EphA3 receptor mediate ligand binding and receptor dimerization. J Biol. Chem. 273:20228-20237.
Lawrenson,I.D., S.H.Wimmer-Kleikamp, P.Lock, S.M.Schoenwaelder, M.Down, A.W.Boyd, P.F.Alewood, and M.Lackmann. 2002. Ephrin-A5 induces rounding, blebbing and de-adhesion of EphA3- expressing 293T and melanoma cells by CrkII and Rho-mediated signalling. J Cell Sci 115:1059-1072.
Luo,H., X.Wan, Y.Wu, and J.Wu. 2001. Cross-linking of EphB6 resulting in signal transduction and apoptosis in Jurkat cells. J Immunol. 167:1362-1370.
Manes,S., E.Mira, C.Gomez-Mouton, Z.J.Zhao, R.A.Lacalle, and A.Martinez. 1999. Concerted activity of tyrosine phosphatase SHP-2 and focal adhesion kinase in regulation of cell motility. Mol Cell Biol 19:3125-3135.
Mellitzer,G., Q.Xu, and D.G.Wilkinson. 1999. Eph receptors and ephrins restrict cell intermingling and communication. Nature 400:77-81.
Miao,H., E.Burnett, M.Kinch, E.Simon, and B.Wang. 2000. Activation of EphA2 kinase suppresses integrin function and causes focal-adhesion-kinase dephosphorylation. Nat. Cell Biol. 2:62-69.
Nicola,N.A., E.Viney, D.J.Hilton, B.Roberts, and T.Wilson. 1996. Molecular cloning of two novel transmembrane ligands for Eph-related kinases (LERKS) that are related to LERK-2. Growth Factors 13:141-149.
Ogawa,K., R.Pasqualini, R.A.Lindberg, R.Kain, A.L.Freeman, and E.B.Pasquale. 2000. The ephrin-A1 ligand and its receptor, EphA2, are expressed during tumour neovascularization. Oncogene 19:6043-6052.
Oh,E.S., H.Gu, T.M.Saxton, J.F.Timms, S.Hausdorff, E.U.Frevert, B.B.Kahn, T.Pawson, B.G.Neel, and S.M.Thomas. 1999. Regulation of early events in integrin signaling by protein tyrosine phosphatase SHP-2. Mol Cell Biol. 19:3205-3215.
Saxton,T.M. and T.Pawson. 1999. Morphogenetic movements at gastrulation require the SH2 tyrosine phosphatase Shp2. Proc. Natl. Acad. Sci. U. S. A. 96:3790-3795.
Stolz,D.B., G.Bannish, and B.S.Jacobson. 1992. The role of the cytoskeleton and intercellular junctions in the transcellular membrane protein polarity of bovine aortic endothelial cells in vitro. J. Cell. Sci. 103 (Pt 1):53-68.
Thien,C.B. and W.Y.Langdon. 2001. Cbl: many adaptations to regulate protein tyrosine kinases. Nat Rev. Mol Cell Biol 2:294-307.
Vincent,A.M., J.C.Cawley, and J.Burthem. 1996. Integrin function in chronic lymphocytic leukemia. Blood 87:4780-4788.
Wang,H.U. and D.J.Anderson. 1997. Eph family transmembrane ligands can mediate repulsive guidance of trunk neural crest migration and motor axon outgrowth. Neuron 18:383-96.
Wang,H.U., Z.F.Chen, and D.J.Anderson. 1998. Molecular distinction and angiogenic interaction between embryonic arteries and veins revealed by ephrin-B2 and its receptor Eph-B4 Cell 93:741-53.
Wang,X., P.J.Roy, S.J.Holland, L.W.Zhang, J.G.Culotti, and T.Pawson. 1999. Multiple ephrins control cell organization in C. elegans using kinase-dependent and -independent functions of the VAB-1 Eph receptor. Mol Cell 4:903-913.
Wicks,I.P., D.Wilkinson, E.Salvaris, and A.W.Boyd. 1992. Molecular cloning of HEK, the gene encoding a receptor tyrosine kinase expressed by human lymphoid tumour cell lines. Proc Natl Acad Sci USA 89:1611-1615.
Winning,R.S., J.B.Scales, and T.D.Sargent. 1996. Disruption of cell adhesion in Xenopus embryos by Pagliaccio, an Eph-class receptor tyrosine kinase. Developmental Biology 179:309-19
Zou,J.X., B.Wang, M.S.Kalo, A.H.Zisch, E.B.Pasquale, and E.Ruoslahti. 1999. An Eph receptor regulates integrin activity through R-Ras. Proc Natl Acad Sci USA 96:13813-13818.
Angers-Loustau, A., J. F. Cote, et al. (1999). "Roles of protein tyrosine phosphatases in cell migration and adhesion." 77(6): 493-505.
Chiarugi, P., P. Cirri, et al. (2000). "The low M(r) protein-tyrosine phosphatase is involved in Rho-mediated cytoskeleton rearrangement after integrin and platelet-derived growth factor stimulation." J Biol Chem 275(7): 4640-6.
Edwards, J. G., G. Campbell, et al. (1991). "Vanadate inhibits both intercellular adhesion and spreading on fibronectin of BHK21 cells and transformed derivatives." J Cell Sci 98 (Pt 3): 363-8.
Hubbard, S. R. and J. H. Till (2000). "Protein tyrosine kinase structure and function." Annu Rev Biochem 69: 373-98.
Hunter, T. (1995). "Protein kinases and phosphatases: the yin and yang of protein phosphorylation and signaling." Cell 80(2): 225-36.
Kikawa, K. D., D. R. Vidale, et al. (2002). "Regulation of the EphA2 kinase by the low molecular weight tyrosine phosphatase induces transformation." J Biol Chem 277(42): 39274-9.
Larsen, M., M. L. Tremblay, et al. (2003). "Phosphatases in cell-matrix adhesion and migration." Nat Rev Mol Cell Biol 4(9): 700-11.
Lawrenson, I. D., S. H. Wimmer-Kleikamp, et al. (2002). "Ephrin-A5 induces rounding, blebbing and de-adhesion of EphA3- expressing 293T and melanoma cells by CrkII and Rho-mediated signalling." 115(Pt 5): 1059-1072.
Li, L. and J. E. Dixon (2000). "Form, function, and regulation of protein tyrosine phosphatases and their involvement in human diseases." Semin Immunol 12(1): 75-84.
Miao, H., E. Burnett, et al. (2000). "Activation of EphA2 kinase suppresses integrin function and causes focal-adhesion-kinase dephosphorylation." Nat.Cell Biol. 2(2): 62-69.
Nimnual, A. S., L. J. Taylor, et al. (2003). "Redox-dependent downregulation of Rho by Rac." Nat Cell Biol 5(3): 236-41.
Ostman, A. and F. D. Bohmer (2001). "Regulation of receptor tyrosine kinase signaling by protein tyrosine phosphatases." Trends Cell Biol 11(6): 258-66.
Park, S. (2003). "The EphA8 Receptor Phosphorylates and Activates Low Molecular Weight Phosphotyrosine Protein Phosphatase in Vitro." J Biochem Mol Biol 36(3): 288-93.
Raugei, G., G. Ramponi, et al. (2002). "Low molecular weight protein tyrosine phosphatases: small, but smart." Cell Mol Life Sci 59(6): 941-9.
Reynolds, A. R., C. Tischer, et al. (2003). "EGFR activation coupled to inhibition of tyrosine phosphatases causes lateral signal propagation." Nat Cell Biol 5(5): 447-53.
Rhee, S. G., Y. S. Bae, et al. (2000). "Hydrogen peroxide: a key messenger that modulates protein phosphorylation through cysteine oxidation." Sci STKE 2000(53): PE1.
Schlessinger, J. (2000). "Cell signaling by receptor tyrosine kinases." Cell 103(2): 211-25.
Stein, E., A. A. Lane, et al. (1998). "Eph receptors discriminate specific ligand oligomers to determine alternative signaling complexes, attachment, and assembly responses." Genes Dev. 12(5): 667-678.
Wilson, A. K., A. Takai, et al. (1991). "Okadaic acid, a phosphatase inhibitor, decreases macrophage motility." Am J Physiol 260(2 Pt 1): L105-12.
Zantek, N. D., M. Azimi, et al. (1999). "E-cadherin regulates the function of the EphA2 receptor tyrosine kinase." 10(9): 629-638.
Zelinski, D. P., N. D. Zantek, et al. (2001). "EphA2 overexpression causes tumorigenesis of mammary epithelial cells." Cancer Res 61(5): 2301-6.
M Nakamoto, AD Bergemann, "Divers Roles for the Eph Family of Receptor Tyrosine Kinases in Carcinogenesis" Microscopy Research and Technique 59:58-67 (2002).

## Claims

1. An antibody to an ephrin-interacting domain of an EphA3 receptor for use in preventing, delaying or inhibiting tumour cell metastasis whereby the ability of a tumour cell expressing said EphA3 receptor to respond to ephrin binding in a mammal is modulated and cell-contact repulsion between said tumour cell and another cell that expresses said ephrin is enhanced.

2. The antibody for the use according to Claim 1, wherein neovascularization of a tumour is also prevented, inhibited or delayed.

3. The antibody for the use according to Claim 1, wherein said tumour cell is a malignant melanoma cell.

4. The antibody for the use according to Claim 1, wherein the antibody is the IIIA4 monoclonal antibody.

5. The antibody for the use according to Claim 1, wherein the ephrin expressed by said another cell is human ephrin A5.

6. The antibody for the use according to any preceding claim, wherein the mammal is a human.

7. A pharmaceutical composition comprising an antibody directed to an ephrin-interacting domain of an EphA3 receptor together with a pharmaceutically acceptable carrier, diluent or excipient, for use in preventing, delaying or inhibiting tumour cell metastasis by modulating the ability of a tumour cell expressing said EphA3 receptor to respond to ephrin binding in a mammal, whereby cell-contact repulsion between said tumour cell and another cell that expresses said ephrin is enhanced.

8. The pharmaceutical composition for the use of Claim 7, wherein said antibody is an IIIA4 monoclonal antibody.

9. The use of an antibody to an ephrin-interacting domain of an EphA3 receptor in the manufacture of a medicament for use in preventing, inhibiting or delaying tumour cell metastasis by modulating the ability of a tumour cell expressing said EphA3 receptor to respond to ephrin binding in a mammal, whereby cell-contact repulsion between said cell and another cell expressing said ephrin is enhanced.

10. The use according to Claim 9, wherein neovascularization of a tumour is also prevented, inhibited or delayed.

11. The use according to Claim 9, wherein said tumour cell is a malignant melanoma cell.

12. The use according to Claim 9, wherein the antibody is the IIIA4 monoclonal antibody.

13. The use according to Claim 9, wherein the ephrin expressed by said another cell is ephrin A5.

## Patentansprüche

1. Antikörper gegen eine mit Ephrin wechselwirkende Domäne eines EphA3-Rezeptors zur Verwendung bei der Prävention, Verzögerung oder Hemmung von Tumorzellenmetastase, wodurch die Fähigkeit einer Tumorzelle, die den EphA3-Rezeptor exprimiert, bei einem Säugetier auf Ephrinbindung zu reagieren, moduliert wird und die Zellkontaktabstoßung zwischen der Tumorzelle und einer weiteren Zelle, die das Ephrin exprimiert, verstärkt wird.

2. Antikörper zur Verwendung nach Anspruch 1, worin auch die Gefäßneubildung eines Tumors unterbunden, gehemmt oder verzögert wird.

3. Antikörper zur Verwendung nach Anspruch 1, worin die Tumorzelle eine bösartige Melanomzelle ist.

4. Antikörper zur Verwendung nach Anspruch 1, worin der Antikörper ein monoklonaler Antikörper IIIA4 ist.

5. Antikörper zur Verwendung nach Anspruch 1, worin das von der weiteren Zelle exprimierte Ephrin menschliches Ephrin A5 ist.

6. Antikörper zur Verwendung nach einem der vorangegangenen Ansprüche, worin das Säugetier ein Mensch ist.

7. Pharmazeutische Zusammensetzung, die einen auf eine mit Ephrin wechselwirkende Domäne eines EphA3-Rezeptors gerichteten Antikörper zusammen mit einem pharmazeutisch annehmbaren Träger, Verdünner oder Exzipienten umfasst, zur Verwendung bei der Prävention, Verzögerung oder Hemmung von Tumorzellenmetastase durch das Modulieren der Fähigkeit einer Tumorzelle, die den EphA3-Rezeptor exprimiert, bei einem Säugetier auf Ephrinbindung zu reagieren, wodurch die Zellkontaktabstoßung zwischen der Tumorzelle und einer weiteren Zelle, die das Ephrin exprimiert, verstärkt wird.

8. Pharmazeutische Zusammensetzung zur Verwendung nach Anspruch 7, worin der Antikörper ein monoklonaler Antikörper IIIA4 ist.

9. Verwendung eines Antikörpers gegen eine mit Ephrin wechselwirkende Domäne eines EphA3-Rezeptors zur Herstellung eines Medikaments zur Verwendung bei der Prävention, Hemmung oder Verzögerung von Tumorzellenmetastase durch das Modulieren der Fähigkeit einer Tumorzelle, die den EphA3-Rezeptor exprimiert, bei einem Säugetier auf Ephrinbindung zu reagieren, wodurch die Zellkontaktabstoßung zwischen der Tumorzelle und einer weiteren Zelle, die das Ephrin exprimiert, verstärkt wird.

10. Verwendung nach Anspruch 9, worin auch die Gefäßneubildung eines Tumors unterbunden, gehemmt oder verzögert wird.

11. Verwendung nach Anspruch 9, worin die Tumorzelle eine bösartige MelanomZelle ist.

12. Verwendung nach Anspruch 9, worin der Antikörper ein monoklonaler Antikörper IIIA4 ist.

13. Verwendung nach Anspruch 9, worin das von der weiteren Zelle exprimierte Ephrin das Ephrin A5 ist.

## Revendications

1. Anticorps dirigé contre un domaine d'interaction avec l'éphrine d'un récepteur EphA3, destiné à être utilisé pour prévenir, retarder ou inhiber la métastase de cellules tumorales, moyennant quoi l'aptitude d'une cellule tumorale exprimant ledit récepteur AphA3 à répondre à la liaison de l'éphrine chez un mammifère est modulée et la répulsion de contact cellulaire entre ladite cellule tumorale et une autre cellule qui exprime ladite éphrine est améliorée.

2. Anticorps destiné à être utilisé selon la revendication 1, où la néovascularisation d'une tumeur est également prévenue, inhibée ou retardée.

3. Anticorps destiné à être utilisé selon la revendication 1, où ladite cellule tumorale est une cellule de mélanome malin.

4. Anticorps destiné à être utilisé selon la revendication 1, où l'anticorps est l'anticorps monoclonal IIIA4.

5. Anticorps destiné à être utilisé selon la revendication 1, où l'éphrine exprimée par ladite autre cellule est l'éphrine A5 humaine.

6. Anticorps destiné à être utilisé selon l'une quelconque des revendications précédentes, où le mammifère est un humain.

7. Composition pharmaceutique comprenant un anticorps dirigé contre un domaine d'interaction avec l'éphrine d'un récepteur EphA3 avec un véhicule, un diluant ou un excipient pharmaceutiquement acceptable, destinée à être utilisée pour prévenir, retarder ou inhiber la métastase de cellules tumorales en modulant l'aptitude d'une cellule tumorale exprimant ledit récepteur AphA3 à répondre à la liaison de l'éphrine chez un mammifère, moyennant quoi la répulsion de contact cellulaire entre ladite cellule tumorale et une autre cellule qui exprime ladite éphrine est améliorée.

8. Composition pharmaceutique destinée à être utilisée selon la revendication 7, dans laquelle ledit anticorps est un anticorps monoclonal IIIA4.

9. Utilisation d'un anticorps dirigé contre un domaine d'interaction avec l'éphrine d'un récepteur EphA3 dans la fabrication d'un médicament destiné à être utilisé pour prévenir, inhiber ou retarder la métastase de cellules tumorales en modulant l'aptitude d'une cellule tumorale exprimant ledit récepteur AphA3 à répondre à la liaison de l'éphrine chez un mammifère, moyennant quoi la répulsion de contact cellulaire entre ladite cellule et une autre cellule exprimant ladite éphrine est améliorée.

10. Utilisation selon la revendication 9, dans laquelle la néovascularisation d'une tumeur est également prévenue, inhibée ou retardée.

11. Utilisation selon la revendication 9, dans laquelle ladite cellule tumorale est une cellule de mélanome malin.

12. Utilisation selon la revendication 9, dans laquelle l'anticorps est l'anticorps monoclonal IIIA4.

13. Utilisation selon la revendication 9, dans laquelle l'éphrine exprimée par ladite autre cellule est l'éphrine A5.
